# EUROPEAN PATENT APPLICATION

(11) **EP 4 840 878 A1**
(43) Date of publication of application: **18.11.2026**
(21) Application number: 25176654.9
(22) Date of filing: 15.05.2025
(51) Int. Cl.: C07K 14/47, A61K 38/00, C07K 14/705, C07K 14/195

(54) **ARTIFICIAL MEMBRANE COMPARTMENTS**

(71) Applicant: Max-Planck-Gesellschaft zur Förderung der Wissenschaften e.V., 80539 München (DE)
(72) Inventor: Hoffmann, Patrick, 69469 Weinheim (DE); Beck, Martin, 60438 Frankfurt (DE)
(74) Representative: Kador & Partner Part mbB

(57) **Abstract**

The present invention relates to a system comprising a component A comprising a membrane anchor, and a component B comprising a multivalent protein or a fragment thereof, and a composition comprising the component A and the component B. The present invention further relates to a component A comprising a membrane anchor, and a composition comprising the component A. Furthermore, the present invention relates to a a kit of parts comprising a component A comprising a membrane anchor, and a component B comprising a multivalent protein or a multivalent peptide.

Furthermore, the present invention relates to nucleic acid molecules comprising nucleotide sequences encoding the components and/or compounds or respective compositions comprising the same.

Furthermore, the present invention relates to a cell comprising the system, component A, the nucleic acid molecules or respective compositions comprising the same.

Furthermore, the present invention relates to a method for producing artificial membrane compartments, and to artificial membrane compartments and/or artificial membrane compartment-specific membranes obtainable by said method. Furthermore, the present invention relates to a composition or a pharmaceutical composition comprising the artificial membrane compartments and/or artificial membrane compartment-specific membranes.

In addition, the present invention relates to different uses of the previously mentioned. Finally, the present invention further relates to the artificial membrane compartments and/or artificial membrane compartment-specific membranes or respective compositions comprising the same for use in therapy.

## Description

The present invention relates to a system comprising a component A comprising a membrane anchor, and a component B comprising a multivalent protein or a fragment thereof, and a composition comprising the component A and the component B. The present invention further relates to a component A comprising a membrane anchor, and a composition comprising the component A. Furthermore, the present invention relates to a a kit of parts comprising a component A comprising a membrane anchor, and a component B comprising a multivalent protein or a multivalent peptide.

Furthermore, the present invention relates to nucleic acid molecules comprising nucleotide sequences encoding the components and/or compounds or respective compositions comprising the same.

Furthermore, the present invention relates to a cell comprising the system, component A, the nucleic acid molecules or respective compositions comprising the same.

Furthermore, the present invention relates to a method for producing artificial membrane compartments, and to artificial membrane compartments obtainable by said method. Furthermore, the present invention relates to a composition or a pharmaceutical composition comprising the artificial membrane compartments.

In addition, the present invention relates to different uses of the previously mentioned. Finally, the present invention further relates to the artificial membrane compartments or respective compositions comprising the same for use in therapy.

Eukaryotic cells are compartmentalized into membrane-enclosed and membrane-less organelles to separate biochemical reactions and functions. Membrane-less organelles have an open interface to their surroundings. Their composition, size and biophysical properties is dynamic and straight forward to regulate. Compartmentalization into membrane-less organelles is mediated by multivalent interactions between intrinsically disordered regions (IDRs) of proteins or RNA and their local concentration plays a crucial role in their formation. Well known functional cellular examples are the Phenylalanine-Glycine (FG)-rich phase of the nuclear pore complex (NPC), which contains FG-repeat nucleoporins that form the selective permeability barrier between nucleus and cytoplasm (Frey, S., Richter, R. P. & Görlich, D. FG-rich repeats of nuclear pore proteins form a three-dimensional meshwork with hydrogel-like properties. Science (1979) 314, 815-817 (2006); Schmidt, H. B. & Görlich, D. Transport Selectivity of Nuclear Pores, Phase Separation, and Membraneless Organelles. Trends Biochem Sci 41, 46-61 (2016).), the rRNA processing and ribosome biogenesis compartment, the nucleolus (Montgomery, T. S. H. Comparative cytological studies, with especial regard to the morphology of the nucleolus. J Morphol 15, 265-582 (1898).; Brangwynne, C. P., Mitchison, T. J. & Hyman, A. A. Active liquid-like behavior of nucleoli determines their size and shape in Xenopus laevis oocytes. Proc Natl Acad Sci U S A 108, 4334-4339 (2011).), RNA and protein-rich cytoplasmic P granules in germ cells (Brangwynne, C. P. et al. Germline P Granules Are Liquid Droplets That Localize by Controlled Dissolution/Condensation. Science (1979) 324, 1729-1732 (2009).) or stress granules (Nover, L., Scharf, K.-D. & Neumann, D. Cytoplasmic Heat Shock Granules Are Formed from Precursor Particles and Are Associated with a Specific Set of mRNAs. Mol Cell Biol 9, 1298-1308 (1989).; Wolozin, B. & Ivanov, P. Stress granules and neurodegeneration. Nature Reviews Neuroscience vol. 20 649-666 Preprint at https://doi.org/10.1038/s41583-019-0222-5 (2019).). To better study the principles of biomolecular condensation and to engineer synthetic reaction centers, artificial, photoinducible membrane-less compartments have been developed such as the optoDroplets (Shin, Y. et al. Spatiotemporal Control of Intracellular Phase Transitions Using Light-Activated optoDroplets. Cell 168, 159-171.e14 (2017).), OptoGranules (Zhang, P. et al. Chronic optogenetic induction of stress granules is cytotoxic and reveals the evolution of ALS-FTD pathology. Elife e39578 (2019) doi:10.7554/eLife.39578.001.) or Corelets (Bracha, D. et al. Mapping Local and Global Liquid Phase Behavior in Living Cells Using Photo-Oligomerizable Seeds. Cell 175, 1467-1480.e13 (2018).) system. These artificial systems could be conceived as a mimicry of the naturally occurring membrane-less organelles. Yet, they do not include cellular membranes, and as such, they are fundamentally different from the naturally occurring membrane-separated organelles.

Membrane-enclosed organelles, such as the endoplasmic reticulum, are surrounded by a semipermeable membrane that stably separates microenvironments with different biochemical and biophysical properties. The membrane is permeable to water and certain small molecules, but not to macromolecules. Thus, their composition can be more tightly controlled through dedicated transport machineries. They offer exclusive functionalities, such as the generation of ion gradients across membranes and other strictly membrane-associated functions that are important for cellular signalling, mechano-sensing, protein processing and secretion or the immune response.

While the above-introduced tools that induce and regulate the formation of membrane less-compartments are readily available (Shin, Y. et al. Spatiotemporal Control of Intracellular Phase Transitions Using Light-Activated optoDroplets. Cell 168, 159-171.e14 (2017).; Zhang, P. et al. Chronic optogenetic induction of stress granules is cytotoxic and reveals the evolution of ALS-FTD pathology. Elife e39578 (2019) doi:10.7554/eLife.39578.001.; Bracha, D. et al. Mapping Local and Global Liquid Phase Behavior in Living Cells Using Photo-Oligomerizable Seeds. Cell 175, 1467-1480.e13 (2018).), the artificial generation of membrane-bound organelles is less straight forward. To generate artificial membrane-enclosed organelles that mimic the properties of the respective natural organelles, but with specifically designed membranes, components and functions is of high interest to biotechnological engineering (Reifenrath, M., Oreb, M., Boles, E. & Tripp, J. Artificial ER-derived vesicles as synthetic organelles for in Vivo compartmentalization of biochemical pathways. ACS Synth Biol 9, 2909-2916 (2020).) but has not yet been achieved.

It is the object of the present invention to overcome these issues and provide a system and respective methods for the formation of artificial membrane-enclosed organelles.

The instant invention is based on the surprising finding that the interaction of a membrane anchor and a multivalent protein or multivalent peptide leads to the formation of membrane compartments in cells. It was found that the concept of phase separation may be taken advantage of for generating membrane compartments, i.e. artificial organelles comprising membranes.

This is achieved by providing a component comprising a membrane anchor as means for anchoring the system in cellular membranes. The interaction of this key component with a further component comprising a multivalent protein or a multivalent peptide as means for inducing phase separation, results in the formation of a condensed phase around membranes which drives the formation of separated artificial membrane compartments that can be compositionally engineered. This triggers large-scale dynamic membrane rearrangements and compartmentalization inside cells. The inventive platform technology enables the provision of artificial membrane compartments and further clustering of various membrane protein families within these membrane compartments, allowing their enrichment and identification at specific membrane sites.

The present invention therefore provides a system comprising
a component A comprising a membrane anchor, and
a component B comprising a multivalent protein or a multivalent peptide,
wherein either
   i) the component A is capable to bind to the component B, or
   ii) the component A and the component B form a compound in which component A is bound to component B.

The membrane anchor comprised in component A is not limited and may comprise, or consist of, any compound with membrane binding capacity. Preferably, the membrane anchor comprised in component A is capable of recruiting component B to membranes, preferably to cellular membranes, more preferably to organelle membranes.

Preferably, the membrane anchor comprises, preferably consists of, a lipid binding domain, an amphipathic α-Helix, a hydrophobic transmembrane domain, a lipid anchor, a fatty acid chain, a peripheral membrane protein, an integral membrane protein, a lipid anchored protein, a transmembrane protein or the membrane or transmembrane domains thereof.

A lipid binding domain refers to any domain enabling a protein to bind lipids. In the sense of the present invention the lipid binding domain may specifically refer to lipid head group binding domains. This enables the lipid binding domain to bind to cellular membranes. Preferably, lipid binding domains are selected from the group consisting of PH (pleckstrin homology) Domain, C1 Domain, C2 Domain, FYVE (Fab1, YOTB, Vac1, EEA1) Domain, FFAT (Two Phenylalanines (FF) in an Acidic Tract) Motif, BAR (Bin/Amphiphysin/Rvs) Domain.

Amphipathic α-Helices have both a hydrophobic and a hydrophilic face. The hydrophobic face aligns with the hydrophobic membrane interior and the hydrophilic phase with aqueous environments. This enables a stable, reversible binding to the membrane with high specificity, such as organelle specificity.

A hydrophobic transmembrane domain relates to a transmembrane domain that mostly consists of non-polar amino acids and usually spans the hydrophobic core of a membrane. Transmembrane domains may further comprise hydrophilic amino acids which usually serve a specific purpose, such as forming an ion channel.

Lipid anchors are usually attached to proteins as post-translational modifications. This anchor allows proteins to be stably embedded in the lipid bilayer of a cell's membrane, but the protein does not span the entire membrane compared to transmembrane proteins. Instead, the lipid anchor "attaches" the protein to the membrane by inserting the lipid into the bilayer, essentially "anchoring" the protein to the membrane surface. Preferably, lipid anchors are selected from the group consisting of glycosylphosphatidylin-ositol (GPI) anchors, prenyl anchors, myristoyl anchors or palmitoyl anchors.

GPI anchors consist of complex glycolipids that are usually added to the C-terminus of proteins.

Prenyl anchors usually comprise a farnesyl or geranylgeranyl group. Usually, prenyl anchors are linked to cysteine residues close to the C-terminus of a protein, preferably at CaaX motifs. Usual targets for prenylation are, for example, small GTPases, such as Ras and Rho family GTP-ases, Src family kinases and some G-protein coupled receptors.

Fatty acid chains may, for example, be myristoyl or palmitoyl groups. Myristoyl groups are often linked to N-terminal glycine residues. Palmitoyl groups are usually linked to cysteine residues.

Preferably, in case the membrane anchor of component A is a lipid anchor, component A may further comprise a protein, fragment or motif thereof that is usually post-translationally modified with the lipid-based membrane anchor. Such embodiments refer to membrane anchors being lipid anchored proteins.

More preferably, the membrane anchor comprises, preferably consists of, a lipid binding domain, a peripheral membrane protein, an integral membrane protein, a lipid anchored protein, a transmembrane protein or the membrane or transmembrane domains thereof. Even more preferably the membrane anchor comprises, preferably consists of, a peripheral membrane protein, an integral membrane protein, a lipid anchored protein, a transmembrane protein or the membrane or transmembrane domains thereof. Even more preferably, the membrane anchor comprises, preferably consists of, a transmembrane protein, most preferably the transmembrane domain thereof.

The membrane anchor may preferably comprise, preferably consist of, an organelle specific membrane anchor or a plasma membrane specific membrane anchor. More, preferably the membrane anchor comprises, preferably consists of, an organelle specific membrane anchor.

The membrane morphology of the artificial membrane compartments may be at least to some extent determined by the origin of the underlying native membrane. Hence, ER membranes may preferably be prone to form artificial membrane compartments comprising stacked membrane sheets. Vacuolar, endo-lysosomal and Golgi membranes may result in a vacuolar clustering with stacked membranes or vesicular clustering of artificial membrane compartments.

Hence, the selection of an organelle specific membrane anchor may enable to engineer the resulting artificial membrane compartments by pre-determining the origin, preferably organelle-specific origin, of the membrane the artificial membrane compartment comprises.

The use of organelle specific membrane anchors may have the further advantage that organelle specific compounds, preferably organelle specific proteins, may be enriched in the artificial membrane compartments.

Preferably, the membrane anchor comprises, preferably consists of, a nucleus specific membrane anchor, an endoplasmic reticulum (ER) specific membrane anchor, vacuole specific membrane anchor, endo-lysosomal membrane anchor, Golgi apparatus specific membrane anchor, chloroplast specific membrane anchor, peroxisome specific membrane anchor, a mitochondria specific membrane anchor, or a plasma membrane specific membrane anchor. More preferably, the membrane anchor comprises, preferably consists of, a nucleus specific membrane anchor, an endoplasmic reticulum (ER) specific membrane anchor, vacuole specific membrane anchor, endo-lysosomal membrane anchor, Golgi apparatus specific membrane anchor, chloroplast specific membrane anchor, peroxisome specific membrane anchor, or a mitochondria specific membrane anchor. Most preferably, the membrane anchor comprises, preferably consists of, an endoplasmic reticulum (ER) specific membrane anchor.

Preferably, the membrane anchor comprises, or preferably consists of, a membrane protein or the membrane or transmembrane domain thereof. More preferably, the membrane anchor comprises, preferably consists of, a membrane protein or the membrane or transmembrane domain thereof selected from an ABC (ATP-binding-cassette) transporter, a transmembrane ATPase, an ion channel, a solute carrier (SLC) transporter, an endoplasmic reticulum (ER)- transmembrane protein, an endo-lysosomal transmembrane protein, a protein translocase, a lipid enzyme, a lipid scramblase, lipid transporter, a plasma membrane surface receptor, a cell adhesion protein, or a G-protein coupled receptor. More preferably, the membrane anchor comprises, preferably consists of, an endoplasmic reticulum (ER)- transmembrane protein or the transmembrane domain thereof. Most preferably, the membrane anchor comprises, preferably consists of, the transmembrane domain of an endoplasmic reticulum (ER)- transmembrane protein.

Preferably, the membrane anchor comprises, preferably consists of, the membrane domain of lanosterol 14-alpha demethylase CYP51, also called Erg11, translocon channel subunit Sec 63, oligosaccharyltransferase complex subunit Ost1, calcium ATPase Pmr1, transient receptor potential (TRP) channel Yvc1, TRP channel TRPV6, CDP-diacylglycerol--inositol 3-phosphatidyltransferase Pis1, phosphatidylethanolamine N-methyltransferase Opi3, ER transmembrane helix translocase Spf1, stretch-activated calcium channel Mid1, mechanosensitive calcium channel Piezo1, ABC transporter Ybt1, ABC transporter Yor1, protein kinase R (PKR)-like endoplasmic reticulum kinase PERK, inositol-requiring enzyme IRE1, ABC transporter CFTR, ABC transporter ABCA1, sodium leak channel NALCN, Inositol-3-phosphate receptor Ins3PR, ryanodine receptor 1-3 RyR1-3, P2X receptor P2X4, P2X receptor P2X7, cell surface receptor PD-L1, glucagon-like peptide-1 receptor GLP1R, parathyroid hormone 1 receptor PTHR1, histamine H1 receptor HRH1, gastric inhibitory polypeptide receptor GIPR, Leucine-rich repeat G-protein coupled receptor 5 LGR5, G-protein coupled receptor family C group 5 member D GPCR5D, cell adhesion protein Claudin-6, cell adhesion protein Claudin-18.2, C-X-C chemokine receptor type 4 CXCR4, C-C chemokine receptor 8 CCR8, sodium voltage-gated channel alpha subunit 9 Naᵥ1.7, SARS-CoV2 Spike-protein, delta opioid receptor DOR, relaxin-like family peptide receptor 1 RXFP1, interleukin-receptor 3 CD123, death receptor 5 DR5, insulin receptor IR, calcium release-activated channels CRAC, stromal interaction molecule 1 STIM1, metalloreductase STEAP2, epidermal growth factor receptor EGFR. Most preferably, the membrane anchor comprises, preferably consists of, the membrane domain of lanosterol 14-alpha demethylase CYP51, also called Erg11.

Preferably, the membrane anchor comprises, preferably consists of, an amino acid sequence selected from SEQ ID 1-26 or an amino acid sequence having a sequence identity with an amino acid sequence selected from SEQ ID 1-26 of at least 80 %, preferably at least 90 %, more preferably at least 95 %. Such amino acid sequence having the sequence identity with an amino acid sequence selected from SEQ ID 1-26 has membrane binding capacity and is thus capable to serve as a membrane anchor. More preferably, the membrane anchor comprises, preferably consists of an amino acid sequence selected from SEQ ID 1-26.

SEQ ID 1 corresponds to the ER membrane anchor CYP51/Erg11 (1-55aa). SEQ ID 2 corresponds to lipid binding C1 domain of PKCbeta (C1a+C1b) (31-158aa). SEQ ID 3 corresponds to an outer mitochondria membrane anchor of Tom20 (1- 52aa). SEQ ID 4 corresponds to Sec63 (yeast). SEQ ID 5 corresponds to Ost1 (yeast). SEQ ID 6 corresponds to Pmr1 (yeast). SEQ ID 7 corresponds to Yvc1 (yeast). SEQ ID 8 corresponds to Pis1 (yeast). SEQ ID 9 corresponds to Opi3 (yeast). SEQ ID 10 corresponds to Spf1 (yeast). SEQ ID 11 corresponds to Mid1 (yeast). SEQ ID 12 corresponds to Ybt1 (yeast). SEQ ID 13 corresponds to Yor1 (yeast). SEQ ID 14 corresponds to Rtn1 (yeast). SEQ ID 15 corresponds to Heh2 (yeast). SEQ ID 16 corresponds to Cystic fibrosis transmembrane conductance regulator (CFTR1) (human). SEQ ID 17 corresponds to ATP-binding cassette transporter A1 (ABCA1) (human). SEQ ID 18 corresponds to Transient receptor potential cation channel subfamily V 6 (TRPV6) (human). SEQ ID 19 corresponds to Sodium leak channel (NALCN) (human). SEQ ID 20 corresponds to Piezo1 (human). SEQ ID 21 corresponds to Inositol3phosphate receptor 3 (Ins3PR3) (human). SEQ ID 22 corresponds to PD-L1 receptor (human). SEQ ID 23 corresponds to Parathyroid hormone receptor 1 (PTH1R) (human). SEQ ID 24 corresponds to Glucagon-like-peptide1 receptor (GLP1R) (human). SEQ ID 25 corresponds to Histamine receptor H1 (HRH1) (human). SEQ ID 26 corresponds to CD9 (human).

More preferably, the membrane anchor comprises, preferably consists of, the amino acid sequence of SEQ ID 1 or an amino acid sequence having a sequence identity with the amino acid sequence of SEQ ID 1 of at least 80 %, preferably at least 90 %, more preferably at least 95 %. Most preferably, the membrane anchor comprises, preferably consists of the amino acid sequence of SEQ ID 1.

As indicated component B comprises a multivalent protein or a multivalent peptide.

Multivalency is defined as the ability of a molecule, such as, e.g. a protein or nucleic acid, to engage in multiple simultaneous interactions through repeated interaction domains or motifs. When multivalent interactions occur together, they can drive phase separation. Phase separation usually leads to the formation of non-membrane bound compartments, such as, e.g. stress granules, P-bodies, or nucleoli.

A multivalent protein or multivalent peptide according to the invention is able to engage in multiple simultaneous interactions. Hence, it comprises, or consists of, repeated interaction domains or motifs. Interaction domains are preferably derived from a multidomain protein or a fragment thereof comprising multiple interaction domains. Interaction motifs are preferably derived from an intrinsically disordered protein (IDP) or an intrinsically disordered region (IDR). An intrinsically disordered protein region (IDR) refers to a part of a protein that lacks a stable, defined three-dimensional structure under physiological conditions. Unlike well-structured proteins or domains that fold into compact, stable shapes, intrinsically disordered regions do not adopt a specific conformation but instead exist in a dynamic, flexible state.

The multivalent protein or multivalent peptide of component B is not limited and may comprise, or consist of, any protein or peptide which has the capacity of forming multiple equal binding sites and inducing phase separation.

Preferably, the multivalent protein or the multivalent peptide of component B comprises, or consists of, a phase separating protein or a phase separating peptide.

The phase separating protein or peptide is not limited and may comprise, or consist of, any phase separating protein or peptide which has the capacity of inducing phase separation. Phase separating proteins or peptides are proteins or peptides that undergo phase separation. They are capable to associate and/or self-associate into a complex by forming a network of bonds, usually a network of non-covalent bonds, leading to a condensed phase. Examples of phase separating proteins or peptides are intrinsically disordered proteins (IDP), intrinsically disordered protein regions (IDR) or multidomain proteins having multiple protein interactions domains.

Preferably, the multivalent protein or the multivalent peptide of component B comprises, or consists of, IDPs, IDRs or multidomain proteins having one or more multiple protein interactions domains. Preferably, the multidomain protein comprise, or consist of, at least two multiple protein interaction domains, more preferably at least three, even more preferably at least four, most preferably at least five multiple protein interaction domains. More preferably the multivalent protein or the multivalent peptide of component B comprises, or consists of, IDPs or IDRs, most preferably comprises, or consists of, IDRs.

Furthermore, it is preferred that the multivalent protein or the fragment thereof is soluble.

Preferably, the multivalent protein or the multivalent peptide comprises, or consists of, fused in Sarcoma (FUS) or fragments thereof, FUSn or fragments thereof, FG-repeats, the heterogeneous nuclear ribonucleoprotein A1 (hnRNPA1) or fragments thereof, or a combination of Small Ubiquitin-like Modifier (SUMO) domains and SUMO interaction motifs (SIM), or a combination of SH3 domains and proline-rich motifs.

Fused in Sarcoma (FUS) is a protein that plays a key role in cellular processes such as RNA metabolism, DNA repair, and cell stress response.

FUSn refers to a nuclear isoform of the FUS protein. The "n" in FUSn stands for "nuclear," indicating that this isoform of the FUS protein is localized in the nucleus of the cell, which is the primary site of its normal activity. FUS (Fused in Sarcoma) itself is a multifunctional RNA-binding protein.

FG repeats are also known as phenylalanine-glycine repeats. FG-repeats are short, repetitive sequences of amino acids found in proteins, particularly of the nucleoporins in the nuclear pore complex (NPC).

SUMO domains refer to the structured domain of the SUMO protein itself. SIMs are short linear motifs in proteins that allow them to bind to SUMO proteins.

SH3 domains and proline-rich motifs are central to protein-protein interactions in many signalling pathways. SH3 domains are small protein modules (about 60 amino acids) found in many signaling and cytoskeletal proteins. Proline-rich motifs are short linear sequences rich in the amino acid proline (P), often found in unstructured regions of proteins.

The multivalent protein or the multivalent peptide may comprise, or consist of, one or more of said proteins or peptides depending on the type and length of protein or peptide used. Preferably, in case of FG-repeats, SUMO domains, SUMO interaction motifs, SH3 domains and proline-rich motifs the multivalent peptide comprises, or consists of, one or more thereof.

Preferably, in case the multivalent protein or the multivalent peptide comprises a combination of SUMO domains and SUMO interaction motifs, the multivalent protein or the multivalent peptide comprises, preferably consists of, at least two SUMO domains and at least two SUMO interaction motifs. Preferably, there is no upper limit for the number of SUMO domains and SUMO interaction motifs, however the number of SUMO domains and SUMO interaction motifs usually does not exceed 20. More preferably, the number of SUMO domains and proline-rich motifs is two to ten SUMO domains and two to ten SUMO interaction motifs.

Preferably, in case the multivalent protein or the multivalent peptide comprises a combination of SH3 domains and proline-rich motifs, the multivalent protein or the multivalent peptide comprises, preferably consists of, at least two SH3 domains and at least two proline-rich motifs. Preferably, there is no upper limit for the number of SH3 domains and proline-rich motifs, however the number of SH3 domains and proline-rich motifs usually does not exceed 20. More preferably, the number of SH3 domains and proline-rich motifs is two to ten SH3 domains and two to ten proline rich motifs.

Preferably, the multivalent protein or multivalent peptide comprises, or consists of, fused in Sarcoma (FUS), FUSn, preferably the low complexity (LC) region of FUS or FUSn, or fragments thereof, or FG-repeats, preferably of nucleoporins. More preferably, the multivalent protein or multivalent peptide comprises, or consists of, FG-repeats of nucleoporins.

Preferably, in case of FG-repeats, preferably of nucleoporins, the multivalent protein or multivalent peptide comprises 10 or more FG-repeats, preferably 15 or more FG-repeats. Preferably, there is no upper limit for the number of FG-repeats, however the number of FG-repeats usually does not exceed 70. Preferably, the number of FG-repeats, preferably of nucleoporins the multivalent protein or multivalent peptide comprises, or consists of 17 to 45 FG-repeats.

Preferably, in case of FG-repeats, preferably of nucleoporins, the multivalent protein or multivalent peptide comprises, preferably consists of, at least 200 amino acids, more preferably at least 250 amino acids, even more preferably 300 amino acids. Preferably, there is no upper limit for the number of amino acids, however the number of amino acids usually does not exceed 1000 amino acids. Preferably, in case of FG-repeats, preferably of nucleoporins, the multivalent protein or multivalent peptide comprises, or consist of, 250 to 800 amino acids, more preferably of 300 to 700 amino acids.

More preferably, the multivalent protein or multivalent peptide comprises, or consists of, the low complexity region of FUS (FUS LC) or the FG-repeats of Nsp1, Nup49, Nup100 or NUP153.

More preferably, the multivalent protein or fragment thereof comprises, or consists of, an amino acid sequence selected from SEQ ID 27-31 or an amino acid sequence having a sequence identity with an amino acid sequence selected from SEQ ID 27-31 of at least 80 %, preferably at least 90 %, more preferably at least 95 %. Such amino acid sequence having said sequence identity with an amino acid sequence selected from SEQ ID 27-31 is able to engage in multiple simultaneous interactions, or has the capacity of inducing phase separation. Even more preferably, the multivalent protein or fragment thereof comprises, or consists of, an amino acid sequence selected from SEQ ID 27-31.

SEQ ID 27 corresponds to the FUS LC region (1-214aa). SEQ ID 28 corresponds to Nsp1 (1-591aa). SEQ ID 29 corresponds to Nup100 (1-570aa). SEQ ID 30 corresponds to hNUP153 (875-1475aa). SEQ ID 31 corresponds to Nup49 (1-249aa).

Even more preferably, the multivalent protein or fragment thereof comprises, or consists of, an amino acid sequence selected from SEQ ID 28-31 or an amino acid sequence having a sequence identity with an amino acid sequence selected from SEQ ID 27-31 of at least 80 %, preferably at least 90 %, more preferably at least 95 %. Even more preferably, the multivalent protein or fragment thereof comprises, or consists of, an amino acid sequence selected from SEQ ID 28-31.

The system provides two options i) or ii).

According to option i), component A is capable to bind component B. In other words, components A and components B are initially present independently of each other but due to the capability of component A to bind to component B, the multivalent protein or multivalent peptide of component B may ultimately bind to the membrane anchor of component A. Hence, component A and component B according to option i) may be seen as precursors. Consequently, component A and component B may assemble into a complex similar to the compound according to option ii). Hence, preferably, component A is initially capable to bind to component B and thus component A and component B are initially precursors or component A is bound to component B. Preferably, this is the case, when component A and component B are expressed in cells.

Alternatively, according to option ii), the component A and component B form a compound in which the multivalent protein or multivalent peptide of component B is bound to the membrane anchor of component A. In other words, the multivalent protein or multivalent peptide of component B may ultimately bind to the membrane anchor of component A are initially linked. Such a linkage may be, e.g. for example a covalent bond or a non-covalent bond. In option ii), preferably component A is fused to component B, more preferably component A is genetically fused to component B. Hence, preferably, the compound in which component A is bound to component B is a fusion protein comprising, or consisting of, component A and component B.

The effect achieved by either of option i) or ii) is that the multivalent protein or the multivalent peptide of component B is recruited to the membranes via the membrane anchor of component A. Furthermore, this recruitment to the membranes results in the formation of membrane compartments in the cell.

According to option ii), since component A and the component B form a compound as defined above, said effect is achieved directly. In other words, the component B is recruited to the membranes directly by binding of the compound comprising component A bound to component B to the membrane via the membrane anchor.

According to option i), due to the capability of component A to bind to component B, upon binding of component A to component B the multivalent protein or multivalent peptide is ultimately recruited to the membrane via the membrane anchor. In other words, the component B is recruited to the membrane by said binding to component A. Further, component A is bound to the membrane via the membrane anchor. The multivalent protein or multivalent peptide of component B is recruited to the membrane by the capability of component A to bind component B.

Option i), i.e. component A is capable to bind component B is preferred. The advantage of the system according to option i) is that the binding of component A to component B may not be permanent resulting in a more flexible system. Furthermore, the system may be adapted depending on the exact type of multivalent protein, multivalent peptide or membrane anchor used. Furthermore, in the system according to option i) the binding of component A to component B may optionally be inducible.

In one exemplary preferred embodiment, the system comprises component A comprising a membrane anchor as defined above, and a component B comprising a multivalent protein or a multivalent peptide as defined above, wherein the component A is capable to bind to the component B.

Preferably, the system is inducible. The term inducible refers to the system being initially not active but being turned on and optionally off, in response to a specific stimulus. The stimulus can be given by any means which induces the particular system. Preferably, the stimulus is selected from the group consisting of chemicals, light, temperature changes or cellular stress, preferably chemicals or light, more preferably light.

The system may be preferably inducible by expressing component A and/or component B or the compound under the control of an inducible expression system. Furthermore, the system may be preferably inducible by designing component A and/or component B such that the binding of component A to component B is inducible. A combination thereof is also possible.

Preferably, the expression of component A and/or component B or the compound formed therefrom is inducible, more preferably reversibly inducible.

Further, in case of option i) the binding of component A to component B is preferably inducible, more preferably reversibly inducible.

As defined above, component A and component B according to option i) may be seen as precursors. Consequently, component A and component B may assemble into a complex similar to the compound according to option ii). Hence, preferably, component A is initially capable to bind to component B and thus component A and component B are initially precursors and, upon induction of the binding, component A is bound to component B.

The binding of component A to component B being inducible may be particularly advantageous, since it provides a more flexible system allowing the formation of artificial membrane compartments which can be externally controlled and tightly monitored. Furthermore, an excessive uncontrolled formation of artificial membrane compartments may thereby be prevented.

The term reversibly inducible preferably refers to systems being switched on in the presence of the stimulus but only staying active as long as the stimulus is maintained. Preferably, upon withdrawal of the stimulus, the system is turned off.

Preferably, in option (i) or (ii) component A and preferably component B further comprise one or more protein interaction domains (PID), wherein the PIDs of component A are ligands of the PIDs of component B. In other words, PIDs of component A and the PIDs of component B are cognate partners. This is particularly preferred for option i) of the inventive system, wherein the component A is capable to bind to the component B. Hence, more preferably in option (i) component A and preferably component B further comprise one or more PIDs, wherein the PIDs of component A are ligands of the PIDs of component B. In case only component A further comprise one or more PIDs, such PIDs are ligands of the PIDs of the multivalent protein of component B. However, preferably also component B further comprises one or more PIDs which are not part of the multivalent protein. Hence, even more preferably, in option (i) component A and component B further comprise one or more PIDs, wherein the PIDs of component A are ligands of the PIDs of component B.

A ligand is a substance that can specifically bind to a target protein, for example a receptor. The binding of the ligand is usually reversible and is made possible in particular by ionic bonds, hydrogen bonds, van der Waals forces hydrophobic effects π-π interactions, dipole-dipole interactions, metal ion coordination, cat-ion-π interactions, and/or hydration effects. The affinity of a ligand to the target protein can be determined using ligand binding assays.

The advantage of the use of PIDs in option i) is that the binding of component A to component B may be facilitated. By the selection of cognate PIDs for component A and component B, more efficient and specific binding of component A to component B is achieved. Furthermore, the use of PIDs may preferably provide an inducible system, wherein preferably the binding of component A to component B is inducible.

The PIDs of component A and/or component B are not particularly limited. Any PIDs may be used, as long as binding between the PIDs of component A and component B is achieved. The PIDs of component A and/or component B may be selected from all typical classes of PIDs and their respective cognate partners, for example PIDs binding to phosphorylated residues, such as SH2 domains, PTB domains, 14-3-3 domains, WW domains, BRCT domains and LIM domains; PIDs binding to proline-rich motifs, such as SH3 domains, WW domains, EVH1 domains and PDZ domains; scaffold and complex building domains, such as PDZ do-mains, LIM domains, WD40 domains; ubiquitin and ubiquitin-like binding domains, such as UBA domains and UIM domains); calcium and metal-ion binding domains, such as EF-Hand domains and C2 domains and allosteric regulation domains; and protein dimerizing domains. The PIDs of component A and component B may be similar or different, i.e. no cognate partners to the PIDs of the multivalent protein or multivalent peptide. Preferably, the PIDs of component A and component B are different, i.e. no cognate partners to the PIDs of the multivalent protein or multivalent peptide.

Preferably, the PIDs of component A and/or component B are inducible, preferably reversibly inducible. The stimulus can be given by any means which induces the particular system. Preferably, the stimulus is selected from the group consisting of chemicals, light, temperature changes or cellular stress, preferably chemicals or light, more preferably light.

Preferably, the PIDs of component A and component B comprise protein dimerizing domains of a protein dimerizing system. More preferably, the PIDs of component A and component B comprise inducible protein dimerizing domains, preferably reversibly inducible protein dimerizing domains. Even more preferably, the PIDs of component A and component B comprise light-inducible or chemically-inducible protein dimerizing domains. Such protein dimerizing systems are known in the art and include for example light-inducible (optogentic) or chemically-inducible dimerizing systems such as the CRY2-CIB1 System, the PhyB-PIF System, the iLID System (improved Light-Induced Dimer), the Abscisic Acid (ABA) System, the Gibberellin (GA) System, the Trimethoprim (TMP) System, or the Rapalog Systems.

Light-inducible dimerizing domains may preferably comprise one or more similar or different proteins responsive to at least one wavelength of light, preferably a wavelength of light in the near UV, visible or infrared regions, more preferably a wavelength from 350 nm to 800 nm.

Preferably, the PIDs of component A and component B are protein dimerizing domains of the CRY2-CIB1 System, the PhyB-PIF System, the iLID System (improved Light-Induced Dimer), the Abscisic Acid (ABA) System, the Gibberellin (GA) System, the Trimethoprim (TMP) System, the Rapalog Systems, the heterodimerisation Halo-X-SNAP system, or the SpyTag-SpyCatcher system. More preferably, the PIDs of component A and component B are protein dimerizing domains of the rapamycin dimerization system, the inducible Light-Induced Dimerization (iLID) dimerizing system, the heterodimerisation Halo-X-SNAP system, or the SpyTag-SpyCatcher system. More preferably, the PIDs of component A and component B are protein dimerizing domains of the inducible Light-Induced Dimerization (iLID) dimerizing system.

The light-sensitive component of the iLID dimerizing system is the engineered light-sensitive protein iLID. iLID consists of a modified LOV2 domain fused at its C terminus to an ssrA peptide. iLID and sspB of the iLID system may be induced by illumination with blue light, preferably having a wavelength of 450 to 500 nm.

The iLID dimerizing system is further characterized in Guntas, G. et al. Engineering an improved light-induced dimer (iLID) for controlling the localization and activity of signaling proteins. Proc Natl Acad Sci U S A 112, 112-117 (2015). https://doi.org/10.1073/pnas.1417910112.

The rapamycin dimerization system is characterized by Haruki, H., Nishikawa, J. & Laemmli, U. K. The Anchor-Away Technique: Rapid, Conditional Establishment of Yeast Mutant Phenotypes. Mol Cell 31, 925-932 (2008). https://doi.org/10.1016/j.mol-cel.2008.07.020.

The Halo-X-SNAP system is characterized in Erhart D, Zimmermann M, Jacques O, Wittwer MB, Ernst B, Constable E, Zvelebil M, Beaufils F, Wymann MP. Chemical development of intracellular protein heterodimerizers. Chem Biol. 2013 Apr 18;20(4):549-57. https://doi.org/10.1016/j.chembiol.2013.03.010.

And the SpyTag-SpyCatcher system is characterized in B. Zakeri, J.O. Fierer, E. Celik, E.C. Chittock, U. Schwarz-Linek, V.T. Moy, & M. Howarth, Peptide tag forming a rapid covalent bond to a protein, through engineering a bacterial adhesin, Proc. Natl. Acad. Sci. U.S.A. 109 (12) E690-E697, (2012). https://doi.org/10.1073/pnas.1115485109.

Preferably, the PIDs of component A are iLID and the PIDs of component B are sspB of the iLID dimerization system.

The induction time may be selected to control the number and appearance of artificial membrane compartments formed by the system. First, the number of observations of artificial membrane compartments may increase with induction time. After exceeding a certain induction time, the total number of observations of artificial membrane compartments may decrease, while the size of stacked artificial membrane compartments may increase.

Preferably, induction is performed for at least 1 s, more preferably at least 10 s, even more preferably at least 30 s, most preferably at least 1 min. There is no upper limit for the induction time. The induction time can usually be very short in the range of seconds or minutes, but can also be several days, as along as the cells are healthy. Usually, the induction time is up to 1 week. Preferably, the induction time is up to 48 h, more preferably 24 h, even more preferably 12 h, even more preferably 6 h, even more preferably 1 h, even more preferably 30 min, even more preferably up to 10 min, most preferably up to 5 min. In case of the iLID system, the induction is performed by illumination and the illumination time preferably takes from 1 s to 48 h, more preferably from 1 s to 24 h, even more preferably from 1 s to 12 h, even more preferably from 1 s to 6 h, even more preferably from 1 s to 1 h, even more preferably from 1 s to 30 min, even more preferably from 10 s to 10 min, most preferably from 10 s to 3 min.

Preferably, to increase the concentration of component B at the membrane, the number of PIDs contained in component A and/or B may be increased. Increased local concentration of component B bound to the membrane surface leads to increased membrane coalescence and favours the formation of artificial membrane compartments. Increasing the number of PIDs in component A and/or B may be particularly advantageous, since this may enable a broader variety of compounds to be used as component A and/or B. For example, increasing the number of PIDs may compensate for a multivalent protein contained in component B having a lower potency to drive phase separation. In such an example, a lower potency of the multivalent protein or fragment thereof may be compensated by the higher concentration thereof at the membrane due to increased number of PIDs.

Preferably, the component A and/ or component B comprises at least one PID. There is no upper limit for the number of PIDs, however usually the number of PIDs does not exceed 10, more preferably 5. Even more preferably, component A and/or component B comprises between one and three PIDs.

If the iLID dimerizing system is used, it is preferred that component A comprises at least one, more preferably between one and three iLID domains and component B comprises one sspB.

In one exemplary preferred embodiment, the system comprises component A comprising a membrane anchor as defined above and one or more PIDs, and a component B comprising a multivalent protein or a multivalent peptide as defined above and one or more PIDs, wherein the component A is capable to bind to the component B and wherein the PIDs of component A are ligands of the PIDs of component B.

Preferably, component A and/or component B further comprise an organelle localization signal (OLS). Preferably, the OLS is selected from an ER localization signal (ELS) or a nuclear localization signal (NLS). More preferably, the NLS is Gallus gallus ferritoid, the ELS is CYP51. The presence of an OLS allows controlling the localization of component A and or component B within a predetermined organelle. Thereby, the origin of the membrane for the artificial membrane compartment may be determined.

Preferably, component A and/or component B further comprise a tag. It may be any affinity tag or fluorescent tag known in the art. Preferably the tag is a fluorophore. Preferably, in case both components are tagged, the tags of component A and component B are different. Even more preferably component A comprises GFP and/or component B comprises mCherry. The tag may be fused, preferably genetically fused, to component A and/or component B. It may, for example, be used for monitoring the formation of the artificial membrane compartments and the tracking of the labelled components during formation and within the artificial membrane compartments.

Preferably, component A and/or component B further comprise a cleavage site between the tag and the membrane anchor or the multivalent protein or the multivalent peptide. This enables removal of the tag from the artificial membrane compartments.

Preferably, the system further comprises a component C, wherein component C comprises, or consists of, proteins, RNAs, lipids, or small molecules. Component is not limited and may comprise any protein, RNA, lipid or small molecule. In particular, component C may comprise any protein, RNA, lipid or small molecule of interest to be enriched in the artificial membrane compartment. Preferably, component C comprises, or consists of, one or more proteins. More preferably, component C comprises, or consist of, membrane proteins.

Preferably, component C comprises, or consists of, lanosterol 14-alpha demethylase CYP51, translocon channel subunit Sec 63, oligosaccharyltransferase complex subunit Ost1, calcium ATPase Pmr1, transient receptor potential (TRP) channel Yvc1, TRP channel TRPV6, CDP-diacylglycerol-inositol 3-phosphatidyltransferase Pis1, phosphatidylethanolamine N-methyltransferase Opi3, ER transmembrane helix translocase Spf1, stretch-activated calcium channel Mid1, mechanosensitive calcium channel Piezo1, ABC transporter Ybt1, ABC transporter Yor1, protein kinase R (PKR)-like endoplasmic reticulum kinase PERK, inositol-requiring enzyme IRE1, ABC transporter CFTR, ABC transporter ABCA1, sodium leak channel NALCN, Inositol-3-phosphate receptor Ins3PR, ryanodine receptor 1-3 RyR1-3, P2X receptor P2X4, P2X receptor P2X7, cell surface receptor PD-L1, glucagon-like peptide-1 receptor GLP1R, parathyroid hormone 1 receptor PTHR1, histamine H1 receptor HRH1, gastric inhibitory polypeptide receptor GIPR, Leucine-rich repeat G-protein coupled receptor 5 LGR5, G-protein coupled receptor family C group 5 member D GPCR5D, cell adhesion protein Claudin-6, cell adhesion protein Claudin-18.2, C-X-C chemokine receptor type 4 CXCR4, C-C chemokine receptor 8 CCR8, sodium voltage-gated channel alpha subunit 9 Naᵥ1.7, SARS-CoV2 Spike-protein, delta opioid receptor DOR, relaxin-like family peptide receptor 1 RXFP1, interleukin-receptor 3 CD123, death receptor 5 DR5, insulin receptor IR, calcium release-activated channels CRAC, stromal interaction molecule 1 STIM1, metalloreductase STEAP2, and/or epidermal growth factor receptor EGFR.

More preferably, component C comprises, or consists of, one or more of translocon channel subunit Sec 63, oligosaccharyltransferase complex sub-unit Ost1, calcium ATPase Pmr1, transient receptor potential (TRP) channel Yvc1, CDP-diacylglycerol-inositol 3-phosphatidyltransferase Pis1, phosphatidylethanolamine N-methyltransferase Opi3, and/or ER transmembrane helix translocase Spf1.

Furthermore, it is preferred that either
i) component C is capable to bind to component A and/or B, or
ii) the component C and the component A and/or the component B form a compound in which component C is bound to component A and/or component B.

The explanations given above to option i) and ii) of components A and B equally apply.

Hence, according to option i), component C is capable to bind component A and/or B. In other words, components C and components A and B are initially present independently of each other but due to the capability of component C to bind to component A and/or B, the component C may ultimately bind to component A and/or B. Hence, component A, B and C according to option i) may be seen as precursors.

Alternatively, according to option ii), the component C and component A and/or B form a compound in which component C is bound to component A and/or B. In other words, component C and component A and/or B are initially linked. Such a linkage may be, e.g. for example a covalent bond or a non-covalent bond. In option ii), preferably component C is fused to component A and/or B, more preferably component A is genetically fused. Hence, preferably, the compound in which component C is bound to component A and/or B is a fusion protein comprising, or consisting of, component A, B and C.

As a consequence, component C may be enriched in the artificial membrane compartments formed by the system.

Preferably, the component C comprises, or consist of one or more PIDs which is a cognate partner to the PIDs of component A and/or B. Preferably, the embodiments described above for component A to component B are equally applicable to the PIDs contained in component C to enable its binding to component A or B. Hence, for examples if the PIDs of component A and component B are protein dimerizing domains of the inducible Light-Induced Dimerization (iLID) dimerizing system, preferably the PIDs of component A are iLID and the PIDs of component B are sspB, it may be further preferred that component C comprises an iLID domain.

Preferably, the system of the invention is for producing artificial membrane compartments and/or artificial membrane compartment-specific membranes. In other words, artificial membrane compartment-specific membranes refers to membranes derived from said artificial membrane compartments. Said artificial membrane compartments preferably comprise, or consist of, parallel membranes comprising stacked membrane sheets.

As indicated above, the system enables the formation of a condensed phase around the membranes which drives the formation of separated artificial membrane compartments by dynamic membrane rearrangements and compartmentalization. Usually, said artificial membrane compartments and/or artificial membrane compartment-specific membranes are enriched with component A and component B and comprise lower levels of proteins, preferably membrane proteins, present in the membranes of origin than said membranes of origin. Furthermore, in embodiments comprising the component C, component A, component B and component C are usually enriched in the artificial membrane compartments and/or artificial membrane compartment-specific membranes. In other words, the artificial membrane compartments and/or artificial membrane compartment-specific membranes may usually be characterized by a unique content of proteins, preferably membrane proteins, which differs from naturally occurring organelles or membranes.

Hence, preferably, said artificial membrane compartments and/or artificial membrane compartment-specific membranes are characterized by a unique content of proteins which differs from naturally occurring organelles or membranes. More preferably, said artificial membrane compartments and/or artificial membrane compartment-specific membranes comprise component A and component B, and optionally component C, and/or are depleted of proteins occurring in the membrane of origin.

Preferably, the artificial membrane compartments are produced in cells. The term cell as used herein may refer to primary cells or cell lines. Preferably the cell is a yeast cell or a mammalian cell. Preferably a mammalian cell may be a human cell, murine cell, rat cell, or hamster cell, more preferably a human embryonic kidney cell or Chinese hamster ovary cell, most preferably human embryonic kidney cell.

Furthermore, said artificial membrane compartments are preferably formed at organelles, more preferably at the organelle contact sites, even more preferably at the cell cortex, the nuclear envelope and/or at endoplasmic reticulum.

Preferably, said artificial membrane compartments produced by the inventive system comprise membrane sheets having a distance being 0.25-fold to 4-fold of the stack thickness. More preferably, the artificial membrane sheets have a distance being 0.3-fold to 3-fold, even more preferably 0.5-fold to 2-fold of the stack thickness.

Preferably, the artificial membrane compartments comprise membrane sheets having a distance of 40 to 60 nm and/ or a luminal thickness of 15 to 30 nm measured as described in the experimental section.

As explained above, the key component of the inventive system is component A comprising a membrane anchor which recruits multivalent proteins to the membranes. Hence, the present invention further relates to a component A comprising a membrane anchor, wherein either
i) the component A is capable to bind to a component B comprising a multivalent protein or a fragment thereof, or wherein
ii) the component A and the component B form a compound in which the component A is bound to the component B.

The components and compounds may be in the form of compositions or kit of parts comprising the components and compounds separately or in combination. Accordingly, the invention therefore provides a composition comprising
a component A comprising a membrane anchor, and
a component B comprising a multivalent protein or a multivalent peptide,
wherein either
   i) the component A is capable to bind to the component B, or
   ii) the component A and the component B form a compound in which component A is bound to component B.

Accordingly, the present invention further provides a composition comprising component A comprising a membrane anchor, wherein either
i) the component A is capable to bind to a component B comprising a multivalent protein or a fragment thereof, or wherein
ii) the component A and the component B form a compound in which the component A is bound to the component B.

The invention further provides a kit of parts comprising
i) a component A comprising a membrane anchor, and
ii) a component B comprising a multivalent protein or a multivalent peptide,
wherein either
i) the component A is capable to bind to the component B, or
ii) the component A and the component B form a compound in which component A is bound to component B.

The kit preferably further comprises a means for inducing the system as defined above, preferably for inducing the binding of component A to bind to the component B.

Furthermore, the invention provides nucleic acid molecules comprising nucleotide sequences encoding the components and/or compounds according to the invention. Preferably, nucleic acid molecules comprise plasmids encoding for one or more of the components and/or compounds.

Furthermore, the invention provides a cell comprising the system of the invention, the component A of the invention, or the nucleic acid molecules of the invention.

Preferably, the cell is a eukaryotic cell, preferably a mammalian cell or a yeast cell, as defined above. Preferably, the mammalian is a human cell.

Furthermore, the invention further provides a method for producing artificial membrane compartments comprising introducing the system of the invention, or the component A of the invention, or the kit of parts of the invention, or the nucleic acid molecule of the invention into cells or cell lines, culturing the cells or cell lines to express the components or compounds of the system, or the nucleic acid molecule.

Introducing the system of the invention, or the component A of the invention, or the kit of parts of the invention, or the nucleic acid molecule of the invention into cells or cell lines may be achieved by any method known in the art, preferably as described in the Material and Methods section.

Furthermore, culturing the cells or cell lines to express the components or compounds of the system, or the nucleic acid molecule may be achieved by any method known in the art, preferably as described in the Material and Methods section.

The system according to the invention may preferably be introduced into the cells or cell lines either by co-expressing the components or compounds using plasmid expression or by integrating nucleic acid sequences encoding the components or compounds within the host genome.

Optionally, if the components or compounds of the system are expressed under the control of an inducible expression system, the method further comprises inducing the expression of the components or compounds of the system.

In case the binding of component A and component B is inducible as defined above, such as light-inducible or chemically-inducible, the method preferably further comprises providing a stimulus to induce the binding of component A to component B. In case a light-inducible system is provided, the method preferably further comprises illuminating the cells with light of the required wavelength. In case the iLID dimerizing system is used, the method preferably further comprises illuminating the cells with blue light.

The method preferably further comprises isolating the artificial membrane compartments. The isolation of the artificial membrane compartments may be achieved by any method known in the art, such as microsome subcellular fractionation of lysed cells after artificial membrane compartment induction or preparation of membrane-derived vesicles through chemical or physical initiation of vesicle formation derived from the artificial membrane compartments with enriched membrane proteins. Preferably, isolation of the artificial membrane compartments is achieved by microsome subcellular fractionation of lysed cells after artificial membrane compartment induction or preparation of membrane-derived vesicles through chemical or physical initiation of vesicle formation derived from the artificial membrane compartments with enriched membrane proteins.

Furthermore, the invention provides artificial membrane compartments and/or artificial membrane compartment-specific membranes, obtainable by said method. In other words, artificial membrane compartment-specific membranes refers to membranes derived from said artificial membrane compartments. The artificial membrane compartments and/or artificial membrane compartment-specific membranes are preferably characterized by the features defined above. The artificial membrane compartments may be present in cells or may be isolated therefrom. Preferably, the invention provides isolated artificial membrane compartments and/or the artificial membrane compartment-specific isolated membranes.

The artificial membrane compartments and/or artificial membrane compartment-specific membranes may be comprised in composition or pharmaceutical composition. Hence, furthermore, the invention provides a composition or pharmaceutical composition comprising the artificial membrane compartments and/or artificial membrane compartment-specific membranes.

The system of the invention, the component A of the invention, or the nucleic acid molecules of invention and respective compositions or kits comprising the same are useful for various purposes.

Hence, furthermore, the invention relates to a use of the system according to the invention, the component A according to the invention, or the nucleic acid molecules according to the invention, respective compositions or kits comprising the same for producing artificial membrane compartments, preferably in cells.

Furthermore, the invention relates to a use of the system of the invention, the component A of the invention, or the nucleic acid molecule of the invention, or respective compositions or kits comprising the same for studying and manipulating membrane proteins and membrane associated proteins inside cells.

The present invention in its different aspects is particularly advantageous for these uses, since selected proteins of interest can be clustered in the artificial membrane compartments. This enables structure-function analysis directly inside of cells. Identification and target protein copy numbers are currently some of the biggest bottlenecks for in situ structural biology of membrane protein that can be solved using the present invention.

Preferably, the studying and manipulating comprises determining the structure and/ or the activity of the proteins, and/or monitoring changes, preferably drug induced changes of the structure and/ or the activity of the proteins.

The determining of structure and/or activity of proteins and monitoring changes, preferably drug induced changes thereof may preferably be carried out by cryo-EM, in situ cryo-ET, electron diffraction or similar structure determination methods.

Hence, the system of the invention, the component A of the invention, or the nucleic acid molecule of the invention, or respective compositions or kits comprising the same are particularly useful for studying native lipid-protein interactions, studying native membrane protein folding, stability, activity and sorting into artificial organelles.

Furthermore, the present invention relates to a use of the system of the invention, the component A of the invention, or the nucleic acid molecule of the invention, or respective compositions or kits comprising the same for recruiting and/or enriching proteins, RNAs, lipids, or small molecules, preferably proteins. More preferably the protein are membrane proteins and membrane associated proteins, preferably endogenous membrane resident proteins and/or membrane proteins ectopically expressed.

Furthermore, also the artificial membrane compartments and/or artificial membrane compartment-specific membranes of the invention are useful for various purposes. Hence, the present invention relates to a use of the artificial membrane compartments and/or artificial membrane compartment-specific membranes of the invention or the respective compositions comprising the same for drug delivery to cells.

For this use, artificial membrane compartments and/or artificial membrane compartment-specific membranes may preferably be engineered to contain channels or transporters that facilitate the uptake of drugs, such as therapeutic proteins, nucleic acids or small molecules within them. The synthetic membrane compartments and/or artificial membrane compartment-specific membranes may preferably additionally be enriched for surface receptors to confer cell- or tissue-specificity and used as engineered delivery vehicles to the target.

Furthermore, the invention relates to a use of artificial membrane compartments and/or artificial membrane compartment-specific membranes of the invention or the compositions comprising the same for antigen presentation and/or immunization, preferably to generate therapeutic antibodies against membrane protein targets.

This may be a particularly advantageous approach for antigens which are difficult to purify and present for antibody production. Proteins, such as GPCRs or multi-subunit complexes, are difficult to purify and present for antibody production by conventional approaches such as in detergent micelles, lipid nanodiscs, virus-like-particles or even whole cell presentation for antigen presentation. These classical approaches to generate antibodies often have poor results and currently only few approved therapeutic antibodies against membrane proteins exist.

Enrichment and presentation thereof in the inventive artificial membrane compartments and/or artificial membrane compartment-specific membranes provides an improved platform for antigen presentation of such proteins and enables displaying target membrane protein in native membrane environment.

Furthermore, the invention relates to a use of the artificial membrane compartments and/or artificial membrane compartment-specific membranes of the invention or the compositions comprising the same for assembling multi-subunit membrane protein complexes.

Such use may be of particular relevance in biotechnological applications and metabolic engineering. The artificial membrane compartments and/or artificial membrane compartment-specific membranes can serve as platforms for assembling multi-subunit membrane protein complexes that are difficult to use in isolation and enhance metabolic pathways. Such use preferably includes artificial photosynthesis or synthetic biofuel production in artificial membrane compartments and/or artificial membrane compartment-specific membranes. In these cases, enzymes may preferably be co-enriched in the artificial membrane compartment and/or artificial membrane compartment-specific membranes to optimize efficiency of biochemical reactions or design orthogonal biosynthesis pathways. This could be the modification of lipid synthesis pathways, by enrichment of lipid enzymes in the synthetic membrane compartments and/or artificial membrane compartment-specific membranes.

As such, the artificial membrane compartments and/or artificial membrane compartment-specific membranes could for example be used for the production of anti-inflammatory lipids.

Still further, the artificial membrane compartments and/or artificial membrane compartment-specific membranes are useful in the prevention and/or treatment of diseases. The present invention therefore further provides artificial membrane compartments and/or artificial membrane compartment-specific membranes or pharmaceutical compositions comprising the artificial membrane compartments and/or artificial membrane compartment-specific membranes as an active ingredient for for use in therapy.

Preferably, the use may relate to the delivery of purified artificial membrane compartments and/or artificial membrane compartment-specific membranes to diseased cells, preferably using surface receptors. Furthermore, the use may preferably relate to the expression of artificial membrane compartments with specific membrane proteins directly in diseased cells in order to restore or enhance perturbed cellular functions. For example, the artificial membrane compartments with membrane transporters or ion channels or compositions comprising said artificial membrane compartments may be used to correct ion imbalances in defective cells or to provide clustering of specifically enriched receptors to enhance defective signalling pathways.

In the present invention, it is to be understood that any embodiment disclosed for the system comprising a component A comprising a membrane anchor, and a component B comprising a multivalent protein or a multivalent peptide is also applicable to all other aspects and embodiments of the present invention, i.e. to component A comprising a membrane anchor, to the kit of parts, to nucleic acid molecules comprising nucleotide sequences encoding the components and/or compounds of the system, to a cell comprising the system, to the method for producing artificial membrane compartments and artificial membrane compartments and/or artificial membrane compartment-specific membranes obtainable by said method and to the composition or pharmaceutical composition comprising said artificial membrane compartments and/or artificial membrane compartment-specific membranes, to the different uses and to artificial membrane compartments and/or artificial membrane compartment-specific membranes or composition for use in therapy.

The present invention is further illustrated by the below methods and examples which refer to the following figures:

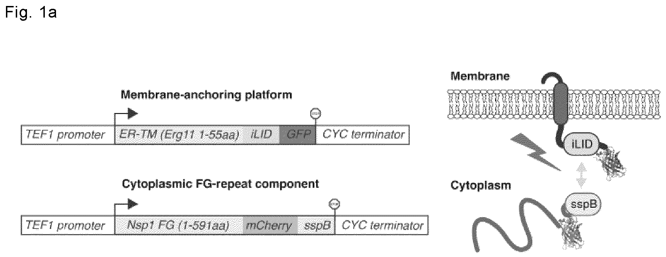

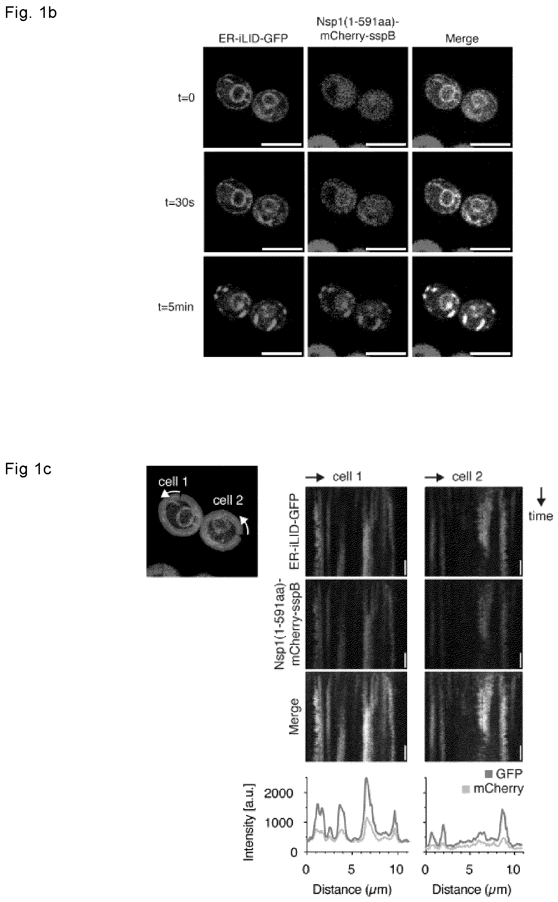

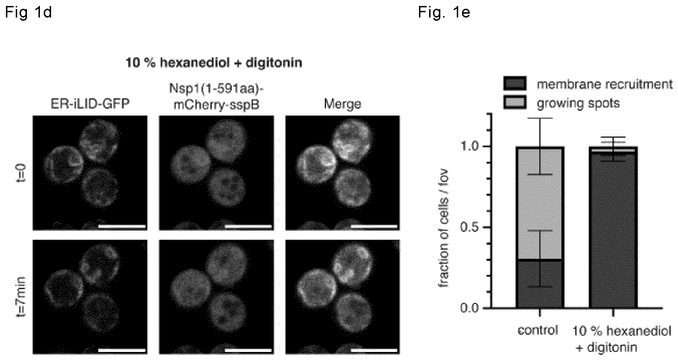

**Figure 1****: Recruitment of FG-repeat domains to ER membrane leads to condensation and coalescence.** (a) FG-repeat domains of yeast Nsp1 (1-591aa) are recruited to the ER membrane using the blue light-inducible dimerization (monitored by mCherry). The iLID-domain is fused to the ER targeting signal and the transmembrane helix of the ER protein Erg11(1-55) (monitored by GFP) (b) Confocal microscopy of S. cerevisiae cells. The ER anchor is localized diffusely in ER and NE membrane at t=0. Nsp1(1-591aa)-FG repeats localize diffusely to both cytoplasm and nucleoplasm. After 30 sec of blue light illumination, Nsp1(1-591aa)-FG repeats are recruited to the ER membrane via the dimerization domain. After 5 min, both construct co-migrate and cluster at several sites in the cell. (c) Kymographs of the fluorescent signals around the cortex of each cell shown in (b) over the course of 5 min. Start point of kymographs is indicated by white arrows. Line profile show the signal along the cortex of the cell at the end of induction. (d) Confocal microscopy of S. cerevisiae cells treated with 10% hexanediol and permeabilized by digitonin. Membrane recruitment of Nsp1(1-591aa)-FG repeats to membrane is triggered by blue light exposure, but no coalescence is observed. (e) Quantification of cells that show coalescence after blue light exposure in each field of view (n = 3). Scale bars: 5 µm in b and d, 1 min (in a, vertical).

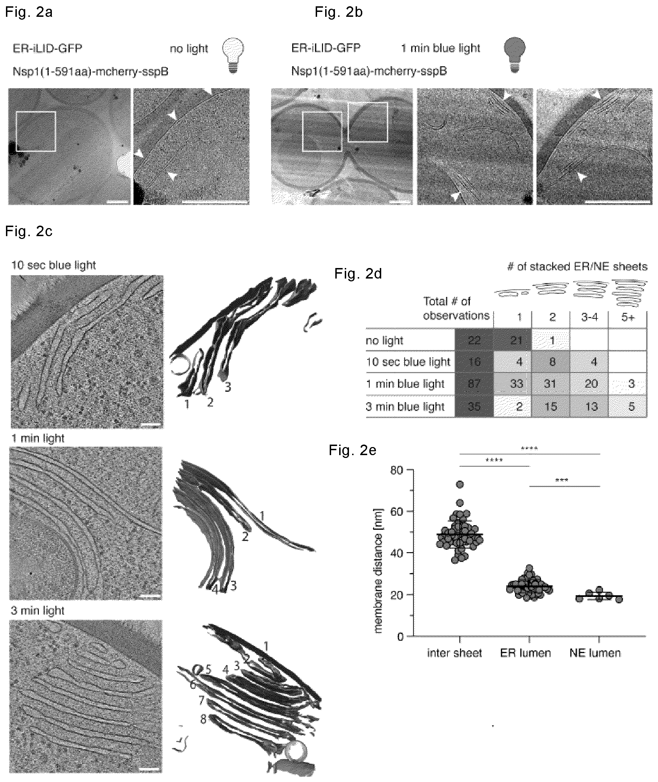

**Figure 2****: Molecular condensation of IDPs on ER membrane results in dynamic stacking of membrane sheets.** (a) Section of a cryo-lamella of yeast cells without blue light induction. Right is a zoomed in view of the orange area. Visible cortical ER and NE are indicated by orange arrows. (b) Section of cryo-lamella of yeast cells after one minute of blue light induction. Zoomed in views of the orange areas are shown separately and regions containing stacked ER at the cell cortex and NE are highlighted with arrows. (c) Representative cryo-ET tomograms and corresponding membrane segmentations of yeast cells after 10 sec, 1 min and 3 min of blue light illumination showing stacked ER membranes. (d) Cryo-tomograms classified by the number of membrane sheets per stacks for different illumination times. (e) Quantification of membrane distances. The mean distance between sheets (inter sheet) is 48.9 nm (SDEV = 6.6 nm, n = 52), the sheet luminal distance (ER lumen) is 24.0 nm (SDEV = 3.2, n = 48) and the NE luminal distance at stacked membranes is 19.4 nm (SDEV = 1.8 nm, n = 6). Scale bars: 1µm in a and b, 50 nm in c. Statistical significance was tested using ANOVA, significance level: *** P< 0.001, **** P< 0.0001.

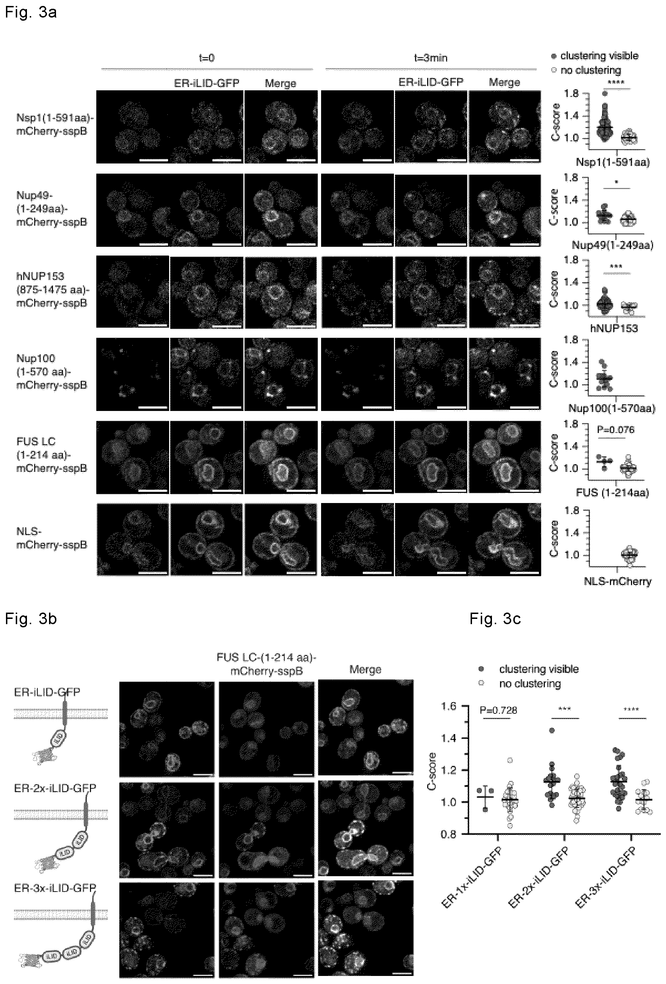

**Figure 3****: Different type of FG-repeat domains and IDRs induce molecular condensation on ER membranes in concentration-dependent manner.** (a) Confocal microscopy of S. cerevisiae cells expressing ER-iLID-GFP anchor and either Nsp1(1-591aa), Nup49 (1-249aa), hNUP153(875-1475aa), Nup100(1-570aa) FG repeats or FUS LC (1-214aa) or NLS mCherry fused to sspB from plasmids. Significant, reversible clustering is observed for Nsp1(1-591aa), Nup49 (1-249aa) and hNUP153(875-1475aa). (b) Confocal microscopy of S. cerevisiae cells expressing FUS LC (1-214aa) and varying numbers of iLID domains at the ER anchor (1x, 2x and 3x) from plasmid. (c) Clustering score for the S. cerevisiae cells expressing FUS LC (1-214aa) and varying numbers of iLID domains at the ER anchor seen in (b). Mean with SDEV is shown and statistical significance was tested using Welch's t test, significance level: * P<0.05, ** P< 0.01, *** P< 0.001, **** P< 0.0001, P values are stated for non-significant results. Scale bars: 5µm.

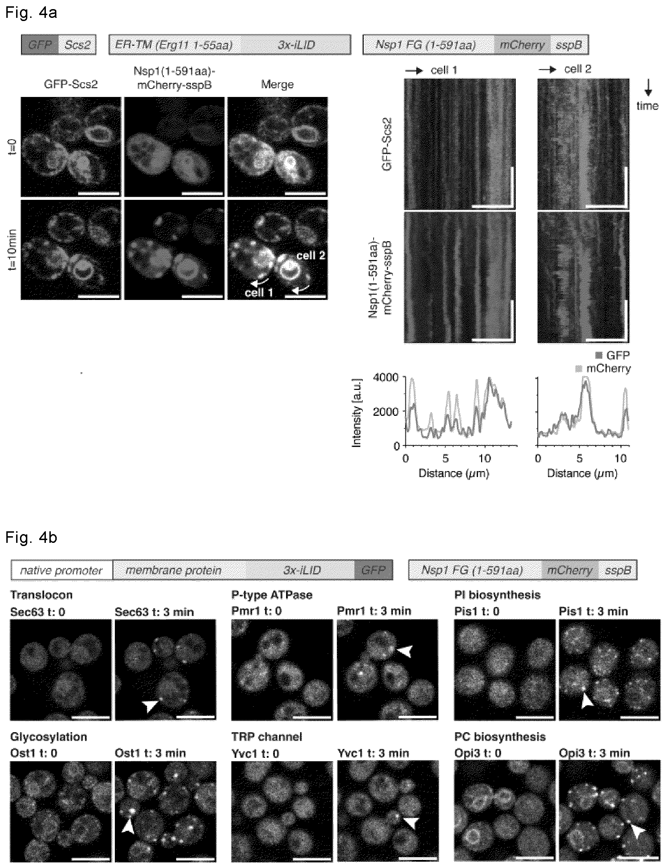

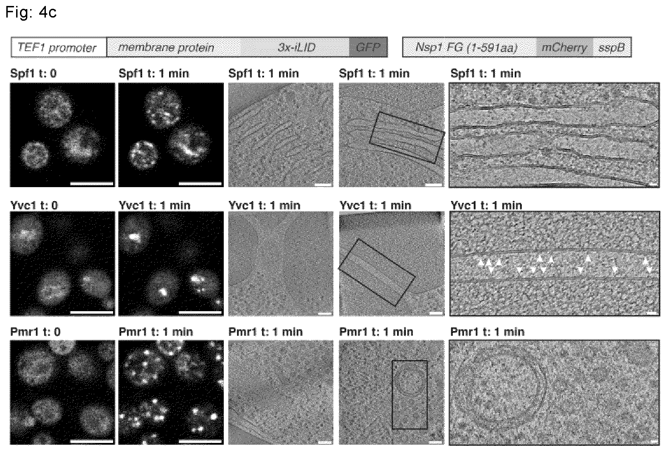

**Figure 4****: IDR driven membrane remodeling can be used to enrich membrane proteins in artificial compartments.** (a) Fluorescence microscopy of cells expressing GFP-Scs2, ER-TM-3xiLID and Nsp1 FG-mCherry-sspB. ER membrane construct without GFP allows for tracking endogenous GFP-labeled Scs2 by fluorescence microscopy. GFP-Scs2 signal does not fully overlap with mCherry positive foci after 10 min (left panels). Right panels show the kymograph of the cortical region of two indicated cells over the course of 10 min and the corresponding fluorescence intensity profile at the end of induction. (b) FM of a selection of different membrane protein that be targeted for inducible clustering and compartmentalization by targeting Nsp1 FG-repeats to their membrane location using the 3x-iLID-sspB membrane stacker system. Arrows indicate sites of clustering after 3 min of blue light induction. (c) FM and cryo-ET overview of induced membrane clustering for cells stably overexpressing Spf1, Yvc1 and Pmr1 using the 3x-iLID-sspB system. Orange boxes marks the zoomed cryo-ET region shown in the right panels. Orange arrowheads indicate visible membrane protein in the Yvc1 overexpression. Scale bars: 5µm in a, b and c (FM) 1 min (in a, vertical) and 100 nm in c (EM overview), 10 nm (EM zoom).

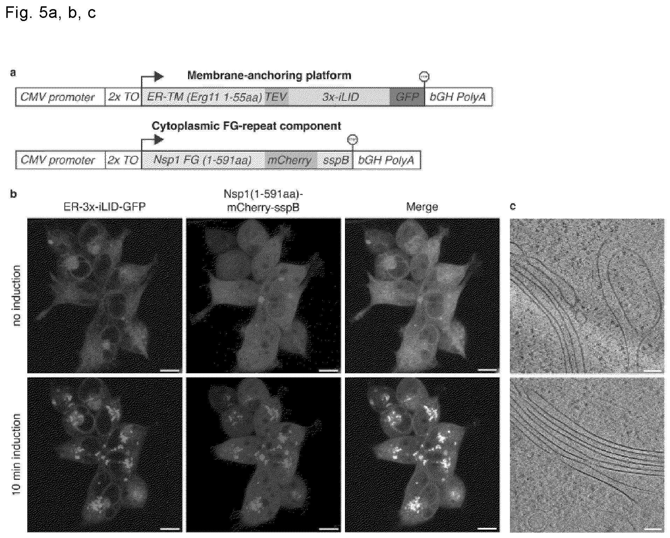

**Figure 5****: Inducible membrane compartmentalization is conserved in HEK293 cells.** (a) Construct design of ER-anchoring platform and IDP component for expression in HEK293 cells. Genes were expressed using a bicistronic vector and stably integrated using the Flp-In T-REx. (b) Confocal microscopy slice of stable HEK293 cells expressing ER-TM-3xiLID-GFP and Nsp1 FG(1-591aa)-mCherry-sspB at the beginning of blue light induction and after 10 min of induction. (c) Cryo-ET slice showing stacked membranes in HEK293 cells expressing ER-TM-3xiLID-GFP and Nsp1 FG(1-591aa)-mCherry-sspB which were plunge frozen after 5 min of blue light induction. Scale bars: 10 µm in b and 100 nm in c.

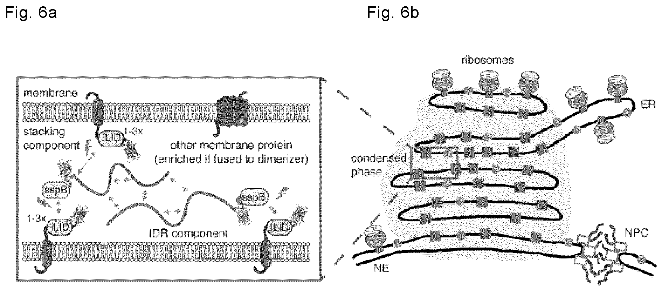

**Figure 6****: Model and mechanism for membrane stacker to induce synthetic membrane compartments.** (a) Model how IDRs generate a condensed phase based on photoinducible dimerization and multivalent interactions between two membranes using the membrane stacker system. (b) Membrane stacker generates synthetic membrane compartment with defined spacing, that is distinctly enriched for specific membrane proteins, while larger cellular components, such as ribosomes and nuclear pore complexes, are excluded.

### Examples

### Material and Methods

### Molecular biology and construct generation

### Yeast membrane stacker system:

pRS316 and pRS313 plasmids containing a TEF promotor and CYC terminator served as backbones. The NLS-1xiLID-EGFP sequence was PCR-amplified from pHR-SFFVp-NLS-iLID-EGFP-FTH1 (addgene plasmid #122147) (Bracha, D. et al. Mapping Local and Global Liquid Phase Behavior in Living Cells Using Photo-Oligomerizable Seeds. Cell 175, 1467-1480.e13 (2018).), while the FUSN-mCherry-sspB coding sequence was PCR-amplified from pHR-SFFVp-FUSN::mCherry::sspB (addgene plasmid # 122148) (Bracha, D. et al. Mapping Local and Global Liquid Phase Behavior in Living Cells Using Photo-Oligomerizable Seeds. Cell 175, 1467-1480.e13 (2018).). To generate a membrane-anchored 1xiLID-EGFP construct, the N-terminal sequence of Erg11 corresponding to the N-terminal membrane targeting and transmembrane domain (1-55aa) was amplified from genomic DNA and cloned into the generated p313-prTEF-NLS-1xiLID-EGFP-CYCt using Gibson Assembly (Gibson, D. G. et al. Enzymatic assembly of DNA molecules up to several hundred kilobases. Nat Methods 6, 343-345 (2009).) to generate p313-prTEF-ER-1xiLID-EGFP-CYCt, Erg11(1-55aa) was later referred to as 'ER', due to the ER membrane localization of the constructs. To generate membrane anchors with multiple iLID domains (2xiLID and 3xiLID), the sequence for multiple iLID domains was gene synthesized (Integrated DNA Technologies) and inserted by Gibson Assembly (Gibson, D. G. et al. Enzymatic assembly of DNA molecules up to several hundred kilobases. Nat Methods 6, 343-345 (2009).) to result in p313-prTEF-ER-2xiLID-EGFP-CYCt and p313-prTEF-ER-3xiLID-EGFP-CYCt. Additionally, membrane anchors without EGFP were generated. To design a condensation component, first, the sequence of FUSN::mCherry::sspB was cloned into the p316 backbone to generate p316-prTEF-FUSN-mCherry-sspB-CYCt. Then, to generate the different FG-based condensation components as membrane stacker, the sequence of FUSN was replaced with the genetic sequence of Nsp1(1-591aa), Nup49(1-249aa), Nup100(1-570aa) amplified from yeast genomic DNA, human NUP153 (875-1475aa) amplified from pEGFP(C3)-Nup153 (addgene plasmid # 64268) (Duheron, V., Chatel, G., Sauder, U., Oliveri, V. & Fahrenkrog, B. Structural characterization of altered nucleoporin Nup153 expression in human cells by Thin-Section electron microscopy. Nucleus 5, 601-612 (2014).). NLS from Gallus gallus ferritoid (Bellapadrona, G. & Elbaum, M. Supramolecular protein assemblies in the nucleus of human cells. Angewandte Chemie - International Edition 53, 1534-1537 (2014).) for NLS-mCherry-sspB was introduced by PCR into the p316 backbone. All plasmids were transformed into chemically-competent TOP10 cells and selected with Ampicillin (50 µg/mL, Sigma-Aldrich) for plasmid amplification. Plasmid inserts were confirmed by sequencing (Microsynth). As an alternative to the light-inducible system, the chemically-inducible FRB-FKBP12-rapamycin system (Haruki, H., Nishikawa, J. & Laemmli, U. K. The Anchor-Away Technique: Rapid, Conditional Establishment of Yeast Mutant Phenotypes. Mol Cell 31, 925-932 (2008).) was cloned into the membrane anchored component and into the condensate component by Gibson Assembly (Gibson, D. G. et al. Enzymatic assembly of DNA molecules up to several hundred kilobases. Nat Methods 6, 343-345 (2009).).

### Stable yeast membrane stacker system:

To generate plasmids for the stably-integrated yeast system, the sequences for the membrane-anchored components were amplified by PCR and cloned into pRS306K (P30646) (Taxis, C. & Knop, M. System of centromeric, episomal, and integrative vectors based on drug resistance markers for Saccharomyces cerevisiae. Biotechniques 40, 73-78 (2006).) using classical restriction-ligation cloning using Ascl and Xmal restriction sites. Sequences for the different condensation components were PCR-amplified and cloned into the pRS305H (P30642) using Gibson assembly. Resulting plasmids were confirmed by restriction analysis and sequencing (Microsynth). To generate stable yeast cells, the plasmids were linearized using either Apal alone or Apal together with NgoMIV and transformed them into S288C *S*. *cerevisiae* cells using lithium acetate transformation protocols (Knop, M. et al. Epitope tagging of yeast genes using a PCR-based strategy: More tags and improved practical routines. Yeast 15, 963-972 (1999).; Gietz, R. D. & Schiestl, R. H. High-efficiency yeast transformation using the LiAc/SS carrier DNA/PEG method. Nat Protoc 2, 31-34 (2007).). Clones with stable integrations at the URA3 locus were selected on YPD-G418 plates and at the LEU2 locus on YPD-HYGB plates and expression of the constructs was verified by fluorescence microscopy. Yeast strains with two stable integrations were generated by sequential transformation and selection.

### Endogenous tagging:

To recruit endogenous membrane proteins into membrane stacks, C-terminal tagging plasmids were generated using the pFA6a backbone system using Gibson Assembly. The resulting plasmids pFA6a-EGFP-sspB::His3MX6, pFA6a-1xiLID-EGFP::His3MX6, pFA6a-3xiLID-EGFP::His3MX6 were validated by restriction analysis and sequencing (Microsynth). Endogenous genetic loci of different membrane proteins were C-terminally labeled with the 3xiLID-EGFP sequence plus selection cassette using homologous recombination according to established protocols (Janke, C. et al. A versatile toolbox for PCR-based tagging of yeast genes: New fluorescent proteins, more markers and promoter substitution cassettes. Yeast 21, 947-962 (2004).). To monitor the ER protein Scs2, it was labeled with N-terminal sfGFP using a seamless tagging strategy (Khmelinskii, A., Meurer, M., Duishoev, N., Delhomme, N. & Knop, M. Seamless gene tagging by endonuclease-driven homologous recombination. PLoS One 6, (2011).). Resulting clones were tested for 5' and 3' integration sites using colony PCR and fluorescence expression and localisation was validated.

### Human membrane stacker system

To transfer the membrane stacker system to into human cells, gene-optimized and synthesized sequences were cloned (Gene Art) for the membrane anchored component Erg11(1-55aa)-TEV-3xiLID-EGFP and of the condensate component Nsp1(1-591aa)-mCherry-sspB. For downstream purification purposes a TEV protease cleavage site was included between ER membrane anchor and the C-terminal domains. The optimized sequences were cloned into pcDNA5-FRT-TO plasmid backbones (Invitrogen) under the control of CMV promotor system, with a tetracycline inducible Tet operator system and also containing the bovine growth hormone polyadenylation (bGH-polyA) signal. Using PCR amplification and Gibson Assembly, a single pcDNA5-FRT-TO plasmid containing the genetic sequences for both constructs was cloned that was verified by sequencing (Microsynth) and used to generate stable HEK293 cell lines.

**Table 1: Constructs and plasmids**

| DNA construct | Identifier | Reference |
|---|---|---|
| M22: pHR-SFFVp-NLS-iLID::EGFP::FTH1 | Addgene plasmid #122147 | Bracha et al. 2018 ¹⁰ |
| M23: pHR-SFFVp-FUSN::mCherry::SspB | Addgene plasmid #122148 | Bracha et al. 2018 ¹⁰ |
| p316-prTEF1-NLS-iLID-EGFP-FTH1-CYCt | pPH028 | This study |
| p316- prTEF1-FUSN-mCherry-sspB-CYCt | pPH029 | This study |
| p316-prTEF1-Nsp1(1-591aa)-mCherry-sspB-CYCt | pPH036 | This study |
| p316-prTEF1-Nup100(1-569aa)-mCherry-sspB-CYCt | pPH037 | This study |
| p316- prTEF1-mCherry-sspB-CYCt | pPH047 | This study |
| p316- prTEF1-NLS-mCherry-sspB-CYCt | pPH048 | This study |
| p313-prTEF1-NLS-iLID-EGFP-FTH1-CYCt | pPH045 | This study |
| p313-prTEF1-NLS-iLID-EGFP-CYCt | pPH049 | This study |
| p313-prTEF1-iLID-EGFP-CYCt | pPH050 | This study |
| p313-prTEF1-Erg11(1-55aa)-iLID-EGFP-CYCt | pPH051 | This study |
| p313-prTEF1-Erg11(1-55aa)-NLS-iLID-EGFP-CYCt | pPH052 | This study |
| p313-prTEF1-PKAbeta-C1-iLID-EGFP-CYCt | pPH053 | This study |
| p313-prTEF1-NLS-PKAbeta-C1-iLID-EGFP-CYCt | pPH054 | This study |
| pFA6a-FRB-KanMX6 | Euroscarf: P30578 | Haruki et al. 2008 ³⁸ |
| pFA6a-2xFKBP-HisMX6 | Euroscarf: P30583 | Haruki et al. 2008 ³⁸ |
| pRS306K | Euroscarf: P30646 | Taxis et al. 2006 39 |
| pRS305H | Euroscarf: P30642 | Taxis et al. 2006 39 |
| pRS305H- prTEF1-FUSN-mCherry-sspB-CYCt | pPH067 | This study |
| pRS305H-prTEF1-Nsp1(1-591aa)-mCherry-sspB-CYCt | pPH068 | This study |
| pRS305H-prTEF1-Nup100(1-569aa)-mCherry-sspB-CYCt | pPH069 | This study |
| pRS305H-prTEF1-NLS-mCherry-sspB-CYCt | pPH070 | This study |
| p316-prTEF1-hNUP153(875-1475aa)-mCherry-sspB-CYCt | pPH071 | This study |
| p316-prTEF1-Nup49(1-249aa)-mCherry-sspB-CYCt | pPH072 | This study |
| gBlock: 2xiLID | - | Ordered via IDT |
| p313-prTEF1-Erg11(1-55aa)-2xiLID-EGFP-CYCt | pPH075 | This study |
| p313-prTEF1-Erg11(1-55aa)-3xiLID-EGFP-CYCt | pPH076 | This study |
| pRS306K-prTEF1-Erg11(1-55aa)-2xiLID-EGFP-CYCt | pPH088 | This study |
| pRS306K-prTEF1-Erg11(1-55aa)-3xiLID-EGFP-CYCt | pPH089 | This study |
| p313-prTEF1-Erg11(1-55aa)-3xiLID-CYCt | pPH091 | This study |
| pRS306K-prTEF1-Erg11(1-55aa)-3xiLID-CYCt | pPH092 | This study |
| pFA6a-EGFP-sspB-His3Mx6 | pPH093 | This study |
| pFA6a-3xiLID-EGFP-His3Mx6 pFA6a-1xiLID-EGFP-His3Mx6 | pPH094 pPH108 | This study This study |
| p313-prTEF1-Spf1-3xiLID-EGFP-CYCt | pPH103 | This study |
| p313-prTEF1-Yvc1-3xiLID-EGFP-CYCt | pPH104 | This study |
| p313-prTEF1-Pmr1-3xiLID-EGFP-CYCt | pPH105 | This study |
| p313-prTEF1-Erg11(1-55aa)-2xFKBP12-EGFP-CYCt | pPH106 | This study |
| p316-prTEF1-Nsp1(1-591aa)-mCherry-FRB-CYCt | pPH107 | This study |
| pcDNA5-FRT-TO | Thermo Fisher Scientific | |
| pcDNA3.1 | Thermo Fisher Scientific | |
| Gene fragment: Erg11(1-55aa)-TEV-3xiLID-EGFP | - | Ordered via GeneArt |
| Gene fragment: Nsp1(1-591aa)-mCherry-sspB | - | Ordered via GeneArt |
| pcDNA5-FRT-TO-Erg11(1-55aa)-TEV-3xiLID-EGFP | pPH097 | This study |
| pcDNA5-FRT-TO-Nsp1-mCherry-sspB | pPH098 | This study |
| pcDNA5-FRT-TO-Nsp1-mCherry-sspB_Erg11(1-55aa)-TEV-3xiLID-EGFP | pPH099 | This study |
| pcDNA3.1-Erg11(1-55aa)-TEV-3xiLID-EGFP | pPH100 | This study |

### Yeast cell culture and transformation

Transformation of S288C *S. cerevisiae* strains was done according to lithium acetate transformation protocols (Knop, M. et al. Epitope tagging of yeast genes using a PCR-based strategy: More tags and improved practical routines. Yeast 15, 963-972 (1999).; Gietz, R. D. & Schiestl, R. H. High-efficiency yeast transformation using the LiAc/SS carrier DNA/PEG method. Nat Protoc 2, 31-34 (2007).)) followed by plate selection with appropriate selection conditions. For auxotrophic selection markers, yeast clones were selected with synthetic complete drop out medium with 2% glucose at 30°C lacking tryptophan (SC-trp), histidine (SC-his), uracil (SC-ura) and combinations thereof. Antibiotic selection was performed in YPD medium supplemented with G418 (400 µg/ml, Sigma-Aldrich) or HygromycinB (100µg/ml, Formedium). For fluorescence microscopy and cryo-fixation by plunge freezing, yeast cells were grown liquid cultures at 30°C and 200 rpm and protected from light allowing for at least 2-3 divisions in log-phase prior to microscopy or cryo-fixation.

### Human cell culture and stable cell line generation

Human Flp-In T-REx 293 cells (Invitrogen) cells were grown in Dulbecco's modified Eagle's medium (Thermo Fisher Scientific) with 10% fetal bovine serum and 1x penicillin-streptomycin (Sigma-Aldrich) in a 5% CO₂ humidified incubator at 37°C. Stable cell lines were generated by transfection of pOG44 plasmid and target plasmid DNA at 9:1 ratio using FuGENE6 (Promega) or Lipofectamine3000 (Invitrogen) transfection reagent. Positive isogenic clones were selected over up to four weeks using 100 µg/ml HygromycinB selection (Invitrogen). Expression of the membrane stacker constructs was induced by addition of 0.5 or 1 µg/ml tetracycline for ~24 or ~48 hrs.

### Fluorescence microscopy and induction of synthetic membrane compartments

Fluorescence microscopy imaging was performed with a Leica Stellaris 5 confocal microscope equipped with a white light laser and with an HC PL APO CS2 63x water objective with NA=1.20. Yeast cultures that were grown in liquid media at 30 °C and 200 rpm in log-phase were allowed to settle onto concanavalin A (Con-A) -coated glass coverslips for 5 min directly prior to fluorescence imaging. HEK293 were adhered to glass-bottom dishes (Mattek) prior to imaging and the microscope stage was kept at 37 °C for imaging. EGFP was excited at 489 nm with a laser power between 0.1- 10 % depending if constructs were expressed from plasmid or as stable integrations; EGFP fluorescence emission was detected with a HyD detector between 494 and 592 nm. mCherry was excited at 587 nm with a laser power between 0.1- 10 % depending if constructs were expressed from plasmid or as stable integrations; mCherry fluorescence emission was detected with a HyD detector between 592 and 839 nm. Images were acquired as sequential lines with line averaging and as z-stacks with 7 slices of 0.5 µm distance. Time lapse movies were either acquired as single slice images or z-stacks as Leica image format files as 12-bit greyscale images.

### Fluorescence image analysis and quantification

Fluorescence images for figure display were adjusted in brightness and contrast using Fiji (Schindelin, J. et al. Fiji: an open-source platform for biological-image analysis. Nat Methods 9, 676-682 (2012).). Co-localization of EGFP and mCherry signals at the cell cortex were done by plotting the average cortical signal corresponding of a width of 10 pixel and displaying its change over time as a kymograph using kymograph builder in Fiji. The relative fluorescence intensity over the last minute of induction was plotted as line profiles in GraphPad Prism to visualize extent of spatial overlap between the signals. First, to quantify cells with coalescence, all cells that showed EGFP and mCherry fluorescence (minimum: 3, maximum: 31 cells, median ~20 cells) in the field of view of an equatorial plane of each time-lapse movie were visually classified whether they formed coalescent clusters or not. Then, for all cells the mean cellular EGFP signal, the standard deviation and the coefficient of variation was determined. The coefficient of variation of the first image in the time lapse was used to normalize and the fold-change after 3 min of induction was then used as clustering score. Statistical significance between the visually classified groups was tested using unpaired t test with Welch's correction in GraphPad Prism.

### Cryo-EM sample preparation

EM support grids (Cu grids for yeast, Au grids for human cells, 200 mesh, carbon or SiO₂ foil, R2/2 or R1/4, Quantifoil) were glow discharged with a Pelco easiGlow for 90 sec at 15 mA.

Yeast grids were optionally coated with ConA (1 mg/ml) for prior to plunge-freezing. Exponentially growing yeast cells were grown to an OD600 of 0.7-0.8 while protected from light. A culture volume of 5 µl was added to the foil side of the grid plunge-frozen into liquid ethane after 10-15 sec of backside blotting with filter paper no1 (Whatman) using a custom manual plunging device. The device was fitted with a custom LED setup to induce cells with blue light in the range of 450-500 nm for 10 sec, 1 min or 3 min prior to blotting and plunging. Alternatively, cells were incubated with a 473 nm laser device for 1 min. The laser was filtered using optical filters to have a strength of ~6 mW/cm2, which was comparable to the LED strength and within the range that had been used before for iLID system applications (Guntas, G. et al. Engineering an improved light-induced dimer (iLID) for controlling the localization and activity of signaling proteins. Proc Natl Acad Sci U S A 112, 112-117 (2015).). Stably expressing HEK293 cells were seeded on glow-discharged and Poly-Lysine coated EM grids at a density of 1x10⁵ cells/ ml, allowed to adhere for 22 hrs, then protein expression was induced for ~24 hrs with half media exchange and a final concentration of 0.5 µg/ml tetracycline. Grids were blue light induced with the custom blue light LED setup (450-500 nm) for ~4 min in the dish and within 10-20 seconds transferred into the plunger setup where the sample was illuminated for the last minute prior to plunge freezing in liquid ethane. A volume of 5 µl culture medium was added to the foil side of the grid to prevent drying and ensure homogeneous blotting. Grids with the mammalian cells were blotted for 15-20 sec prior to plunge freezing using the manual plunge freezing device.

Sample thinning was done by cryo-focused ion beam milling using an Aquilos2 FIB/SEM microscope (Thermo Fisher Scientific) as described previously (Hoffmann, P. C. et al. Structures of the eukaryotic ribosome and its translational states in situ. Nat Commun 13, (2022).). In short, samples were coated with organometallic platinum using a gas injection system for ~10 sec and additionally sputter-coated with platinum at 1 kV and 10 mA current for ~10 sec. SEM imaging was performed with 2 - 10 kV and 13 pA current to monitor the overall milling progress. Preparation was performed stepwise with a gallium ion beam at 30 kV, while reducing the current from 500 pA to 30 pA. Final polishing was performed with 30 pA or 10 pA current to reach a target lamellae thickness between 100 - 200 nm. Some lamellae were sputter coated with platinum after polishing for 1 or 2 sec at 1 kV and 10 mA. For some of the samples, the rough milling step to a lamella thickness of approximately 300 nm was carried out in an automated workflow using AutoTEM, while final polishing was then performed manually as described.

### Cryo-CLEM

Clipped grids were imaged with a Leica DM6 cryo-confocal system equipped with 50x dry objective with 0.9 NA. First, overview grid maps were recorded using the LED system. Confocal stacks with the laser were then recorded for individual grid squares that suited for lamella preparation. This was based on fluorescence signal, ice thickness and possible damage to the carbon layer. Fluorophores were excited at 488 nm with 0.5% laser strength or 552 nm with 1.0% laser strength and fluorescence emission was captured at 500-550 nm and 600-650 nm. Stack sizes ranged from 6-15 µm depending on flatness of the grid square with a step size of 0.3 µm. For correlative data acquisition of IDRs recruited to membranes, grids were transferred back into the cryo-confocal microscope after milling for acquisition of confocal stacks of the fine-milled lamellae. The stacks were recorded at 4% and 8% for 488 nm and 552 nm laser strength, respectively. Stack sizes corresponded to ~3 µm with 0.3 µm step size. Slices from these stacks were correlated to low magnification EM lamella maps using the ICY module EC Clem (Paul-Gilloteaux, P. et al. eC-CLEM: flexible multidimensional registration software for correlative microscopies. Nat Methods 14, 102-103 (2017).) based on recognizable features such as cellular autofluorescence, lamella edges or ice contamination in both images. Areas with fluorescence signal were then selected for tilt series acquisition.

### Cryo-ET acquisition

Data were collected at 300 kV on a Titan Krios G4 microscope equipped with a cold FEG, Selectris X imaging filter and Falcon 4 direct electron detector, operated in counting mode (all Thermo Scientific). For each grid, montaged grid overviews were acquired. Then montages of individual lamellae were taken at an intermediate magnification. Tilt series projections were acquired using SerialEM (Mastronarde, D. N. Dual-Axis Tomography: An Approach with Alignment Methods That Preserve Resolution. J Struct Biol 120, 1-10 (1997).) in low dose mode as 4K x 4K movies of 10 frames each and on-the-fly motion-corrected in SerialEM. The magnification for projection images of 53000x corresponded to a pixel size of 2.4 Å. Dose symmetric tilt series acquisition started from the lamella pretilt of -8° and a dose symmetric acquisition scheme with 2° increments grouped by 2, up to a maximum tilt range of +/-56 to 60°. The exposure time was kept constant and the targeted total dose for each tilt series was kept below ~130 e- per Å². The energy slit width was set to 10 eV and the nominal defocus was varied between -2.5 to -5.0 µm for different tilt series. Dose rate on the detector was targeted to be ~3 - 6 e- /px/sec. For some cryo-ET data acquisition sessions, beam shifted acquisition of multiple tomograms using the PACE-tomography plugin (Eisenstein, F. et al. Parallel cryo electron tomography on in situ lamellae. Nat Methods 20, 131-138 (2023).) was used.

### Cryo-ET image processing, membrane segmentation and distance measurements

TS frames were aligned using the IMOD (Mastronarde, D. N. & Held, S. R. Automated tilt series alignment and tomographic reconstruction in IMOD. J Struct Biol 197, 102-113 (2017).: Kremer, J. R. et al. Computer visualization of three-dimensional image data using IMOD. J Struct Biol 116, 71-76 (1996).) alignframes command before correcting for dose exposure as previously described using a Matlab implementation adapted for tomographic tilt series (Wan, W. et al. Structure and assembly of the Ebola virus nucleocapsid. Nature 551, 394-397 (2017).). CTF estimation was done with gCTF (Zhang, K. Gctf: Real-time CTF determination and correction. J Struct Biol 193, 1-12 (2016).). Poor quality projections were removed after visual inspection. The dose-filtered tilt series were then aligned with patch-tracking in IMOD (Mastronarde, D. N. & Held, S. R. Automated tilt series alignment and tomographic reconstruction in IMOD. J Struct Biol 197, 102-113 (2017).: Kremer, J. R. et al. Computer visualization of three-dimensional image data using IMOD. J Struct Biol 116, 71-76 (1996).) and reconstructed as back-projected tomograms with SIRT-like filtering of 10 iterations at a binned pixel size of 9.6 Å. Back-projected tomograms were reconstructed using novaCTF (Turoňová, B., Schur, F. K. M., Wan, W. & Briggs, J. A. G. Efficient 3D-CTF correction for cryo-electron tomography using NovaCTF improves subtomogram averaging resolution to 3.4 Å. J Struct Biol 199, 187-195 (2017).) with the phase flipping option.

Back-projected tomograms from novaCTF at bin4 were denoised using IsoNet (Liu, Y. T. et al. Isotropic reconstruction for electron tomography with deep learning. Nat Commun 13, (2022).). The neural network was trained on 5 tomograms based on the presence of several different membrane features and data quality. For each tomogram 300 subvolumes were extracted. For deconvolution the CTF estimation from gCTF was taken from the zero-tilt image, the signal-to-noise falloff was set to 0.7 and the deconvolution strength to 1. The neural network was trained for 35 iterations and the results were manually inspected for multiple tomograms.

Automated membrane segmentation was done on the IsoNet-denoised tomograms using the tomography workflow in EMAN2 (Chen, M. et al. Convolutional neural networks for automated annotation of cellular cryo-electron tomograms. Nat Methods 14, 983-985 (2017).; Tang, G. et al. EMAN2: An extensible image processing suite for electron microscopy. J Struct Biol 157, 38-46 (2007).). A neural network was trained on a training set consisting of manually segmented 2D slices with different views of membrane bilayers (~10 per tomogram), as well as slices without membrane bilayers as negatives (~20 per tomogram). Neural network training was done over 50 iterations with the learning rate set to 0.0001. The results were manually inspected and applied to all tomograms. For visualization in figures some segmentations were done using MemBrain (Lamm, L. et al. MemBrain: A deep learning-aided pipeline for detection of membrane proteins in Cryo-electron tomograms. Comput Methods Programs Biomed 224, (2022).; Barad, B. A., Medina, M., Fuentes, D., Wiseman, R. L. & Grotjahn, D. A. Quantifying organellar ultrastructure in cryo-electron tomography using a surface morphometrics pipeline. Journal of Cell Biology 222, (2023).). Segmentations were imported into Amira-Avizo software (Thermo Fisher Scientific) for surface generation. Stacks were auto-thresholded to separate membranes from background. Then 'Separate Objects module' was applied to separate multiple membranes into objects. Objects with a small 3D volume were filtered out according to number of voxels and surfaces were generated for the remaining labels. The surface was re-meshed to reduce the number of meshes to 2%. This visually did not reduce the overall meshing quality of the surface. Finally, membranes sheets were exported individually as point clouds (.ply data type). Membrane measurements were done using the surface morphometrics (Barad, B. A., Medina, M., Fuentes, D., Wiseman, R. L. & Grotjahn, D. A. Quantifying organellar ultrastructure in cryo-electron tomography using a surface morphometrics pipeline. Journal of Cell Biology 222, (2023).) pipeline built upon the pycurv script package (Salfer, M., Collado, J. F., Baumeister, W., FernándezBusnadiego, R. & Martinez-Sánchez, A. Reliable estimation of membrane curvature for cryo-electron tomography. PLoS Comput Biol 16, (2020).) to measure each surface triangle. Normals with an angle of below 90° regarding the closest normal on the neighboring sheet were removed. This step was necessary to exclude normals that were not facing each other on opposite sides of the membrane leaflets and to clean up holes in the surface mesh. In addition, triangles which were the closest mesh for more than 100 meshes on the neighboring sheet were also excluded to correct for mismatches in size between neighboring surfaces that would otherwise lead to overestimated membrane distances. Then mean distances were calculated by normalizing the closest mesh distance for each triangle per sheet in nanometers.

### Results

It was initially reasoned, that the recruitment of FG-repeat containing, phase-separating nucleoporins to the nuclear membrane may facilitate its deformation and thus potentially promote NPC biogenesis. To test this hypothesis, the effects of FG-repeat protein recruitment to cellular membranes was assessed. For this, a two-component synthetic platform was designed for the inducible molecular condensation at membranes, more specifically the recruitment of FG-repeats to the endoplasmic reticulum (ER) membrane. As membrane-anchored component, the ER-targeted transmembrane region (1-55 aa) of the fungal P450 protein family member Erg11 (Monk, B. C. et al. Architecture of a single membrane spanning cytochrome P450 suggests constraints that orient the catalytic domain relative to a bilayer. Proc Natl Acad Sci U S A 111, 3865-3870 (2014).) was fused to a light-inducible protein dimerization domain, iLID (Guntas, G. et al. Engineering an improved light-induced dimer (iLID) for controlling the localization and activity of signaling proteins. Proc Natl Acad Sci U S A 112, 112-117 (2015).) and a derivative of the fluorescent reporter protein GFP in *S. cerevisiae* cells (Figure 1a). As expected, the fusion protein showed yeast-specific ER and nuclear envelope (NE) localization by fluorescence microscopy for both plasmid expression and stable integration at the *ura3* locus. As soluble component, the phase-separating FG-repeats of the yeast nucleoporin protein Nsp1 (1-591aa) was fused to the SspB protein and the fluorescent reporter protein mCherry, and co-expressed, again either as plasmid or as stable integration at the *leu2* locus. This construct initially showed dispersed fluorescent signal in both nucleus and cytoplasm (Figure 1b). Illumination with blue light that triggers co-localization of both components via dimerization of their iLID-sspB domains, led to rapid recruitment of Nsp1(1-591aa) FG-repeats to ER and NE membranes. If the FG-repeats were clustered similar to NPC biogenesis, a spotted localization would have been expected. However, within seconds to minutes the co-migrating fluorescent signals for Nsp1(1-591aa) and the membrane anchor grew into multiple, surprisingly large foci proximate to the cell cortex and the NE membrane (Figure 1b). More intense foci were formed by fusion events of multiple smaller foci over time (Figure 1c). These foci resolved within minutes after stopping blue light exposure. To test if foci formation was promoted by multivalent interactions and formation of an FG-rich phase on the ER membrane, *S. cerevisiae* cells were treated with hexanediol that is known to resolve such interactions (Figure 1 d). In support of this idea, Nsp1(1-591aa) FG-repeats were recruited to ER membranes via the dimerization domain subsequent to hexanediol treatment, yet cells were not able to develop growing foci (Figure 1e). The character and dynamics indicate molecular condensation is likely important for reorganizing these fluorescent foci. However, to which degree the membrane and potential membrane remodeling are involved is not directly resolved by fluorescence microscopy.

To better understand if and how membranes are involved in foci formation, the membrane was visualized in their near-native state without perturbation by chemical fixation using cryo-electron tomography (cryo-ET) of S. *cerevisiae* cells expressing both constructs both without and with blue light illumination. The cells were then thinned using cryo-focused ion beam (cryo-FIB) milling and regions containing ER and NE membrane were targeted for cryo-ET acquisition. Cells without blue light induction showed typical yeast ER morphology, a large fraction of cortical ER, with few connections to the nuclear envelope (West, M., Zurek, N., Hoenger, A. & Voeltz, G. K. A 3D analysis of yeast ER structure reveals how ER domains are organized by membrane curvature. Journal of Cell Biology 193, 333-346 (2011).; Prinz, W. A. et al. Mutants Affecting the Structure of the Cortical Endoplasmic Reticulum in Saccharomyces Cerevisiae. The Journal of Cell Biology vol. 150 http://www.jcb.org (2000).) (Figure 2 a). In contrast, blue-light induced cells showed several instances of stacked membranes at the cell cortex, the NE or at ER-mitochondria contact sites (Figure 2 b). Stacked membranes appeared visually similar to ER membranes in control cells and were often connected to classical ER tubules. But while in non-induced cells, the ER was exclusively formed by single sheets or tubes, FG repeat recruitment to the membrane resulted in rapid formation of stacked membranes even after few seconds of blue light induction with a tendency to larger stacks at longer time points (Figure 2 c and d). Although such stacked membranes are natively observed in mammalian cells, they are very untypical for the yeast ER.

Next the dimensions of the induced membrane compartments were quantified. From tomogram segmentations, an average distance of 48.9 nm (SD = 6.6 nm, n = 52) was measured between individual membrane sheets, while the luminal thickness of sheets was consistently on average 24.0 nm (SD = 3.2, n = 48). For stacked sheets that formed on the NE, the first sheet that correspondent to the membrane of the NE, the luminal thickness was slightly narrower with on average 19.4 nm (SD = 1.8 nm, n = 6) than for the rest of the membrane sheets. Overall, the distance between membrane sheets was consistently about twice as much as their luminal distance. The distance between sheets with about 50 nm was in the range that was previously reported as the maximal extension of ~600aa long Nsp1 FG-repeats in a random array grafted to a surface in molecular simulations (Gamini, R., Han, W., Stone, J. E. & Schulten, K. Assembly of Nsp1 Nucleoporins Provides Insight into Nuclear Pore Complex Gating. PLoS Comput Biol 10, (2014).). In said experimental setting however, both involved membrane surfaces are grafted with Nsp1 FG-repeats.

While stacked membranes appeared visually similar to ER membranes, large macromolecular complexes such as ribosomes were largely absent from the formed membranes stacks, possibly indicating exclusion of either the translocon complex or the ribosome itself. In previous studies of membrane contact site proteins, some IDRs in lipid transport proteins, such as OSBP, have been identified to establish an entropic barrier limiting protein density and diffusion at the contact site (Jamecna, D. et al. An Intrinsically Disordered Region in OSBP Acts as an Entropic Barrier to Control Protein Dynamics and Orientation at Membrane Contact Sites. Dev Cell 49, 220-234.e8 (2019).). Whether entropic principles play a role in the exclusion of ribosomes in the context of induced membrane compartments remains to be investigated. At the same time, nuclear pore complexes, large protein complexes which form the FG-rich permeability barrier between the nucleoplasm and cytoplasm were not enriched in the synthetic membrane compartment that formed at the NE. Overall, this data indicates that induction of condensation at the ER membrane surface can result in the rapid formation of a compositionally distinct, artificial membrane compartment.

Intrigued by the rapid remodeling and stacking of membranes by Nsp1 FG-repeat recruitment, it was tested if other intrinsically disordered proteins of the FG-repeat family, as well as model phase separators such as the low complexity (LC) region of the fused in sarcoma (FUS) RNA binding protein, are capable of inducing similar morphological changes. Besides Nsp1-FG-repeats (1-591aa), the FG repeat regions of Nup49 (1-249aa), Nup100 (1-569aa), human NUP153 (875-1475aa) as well as FUS LC (1-214 aa) were tested and compared to an mCherry control. To better quantify the extent of foci formation in cells over-expressing these constructs from plasmids, a clustering score was introduced by monitoring the variance of fluorescent signal over time (see methods). Membrane recruitment of the FG-repeats regions of Nup49 and hNUP153 also led to a large number of cells showing coalescence. In contrast, Nup100 FG-repeat expressing cells often showed punctate signals overlapping with signal for the ER-anchored component already without blue light illumination (Figure 3 a). These puncta could be a result of low level, residual dimerization during growth of the cells and potentially altered disintegration kinetics of the Nup100 FG-repeat foci. In contrast to the FG-repeat protein constructs, FUS LC expressing cells mostly showed membrane recruitment without further clustering or coalescence. It can be argued that the membrane-bound concentration of FUS LC and its multivalency at the membrane surface might be too low to induce coalescence. Therefore, the concentration of membrane-bound FUS LC was increased by increasing the number of dimerization domains (2x/3x iLID) in the membrane-anchored construct (Figure 3 b). Consistent with higher local concentrations, a larger fraction of cells was observed with coalescence at the membrane. The phenotype and clustering in these cases was reminiscent to the one observed when recruiting Nsp1-FG regions (Figure 3 b, c). The process of membrane stacking is thus dependent on the concentration and availability of intrinsically disordered protein regions at the membrane surface. However, it can be induced by multiple different multivalent proteins and the process is generalizable.

The fast dynamics of membrane stacking and exclusion of large membrane-associated complexes is surprising, since the membrane surface involved is a considerable fraction of the total cellular membrane and it shows morphological features that are distinct from other organelles. This led us to further investigate about the origin of these membranes and how smaller ER proteins distribute within these membranes. As a marker for ER-resident proteins, the small integral ER protein Scs2, a VAMP/synaptobrevin-associated protein (VAP) homolog in *S. cerevisiae* involved in the regulation of phospholipid metabolism and distributed in the ER and NE (Kagiwada, S., Hosaka, K., Murata, M., Nikawa, J.-I. & Takatsuki, A. The Saccharomyces cerevisiae SCS2 Gene Product, a Homolog of a Synaptobrevin-Associated Protein, Is an Integral Membrane Protein of the Endoplasmic Reticulum and Is Required for Inositol Metabolism. J Bacteriol 180, 1700-1708 (1998).) was labelled with GFP. To induce synthetic membrane compartments a system without the fluorescent GFP marker was used for the membrane-anchor and instead the development of membrane stacks was followed by mCherry fluorescence of the Nsp1 FG-repeat construct (Figure 4 a). While Scs2 was present at forming membrane compartments, another pool of Scs2 remained at other locations of the cell. From this, it can be concluded that ER membranes are not or at least only partially consumed during stack formation. While Scs2 as resident ER protein is present, it is not enriched within synthetic membrane compartments (Figure 4 a). Also the involvement of lipid droplets was tested by tagging the lipid droplet marker Erg6 with GFP, of vacuolar membranes by using a fluorescent DAG lipid sensor of the protein kinase C (Lučić, I., Truebestein, L. & Leonard, T. A. Novel Features of DAG-Activated PKC Isozymes Reveal a Conserved 3-D Architecture. J Mol Biol 428, 121-141 (2016).; Roma-nauska, A. & Köhler, A. The Inner Nuclear Membrane Is a Metabolically Active Territory that Generates Nuclear Lipid Droplets. Cell 174, 700-715.e18 (2018).) and of the Golgi by using a PH domain fluorescent reporter (Levine, T. P. & Munro, S. The pleckstrin homology domain of oxysterol-binding protein recognises a determinant specific to Golgi membranes. Current Biology 729-739 (1998).). All of these organelles were not consumed within the analyzed time range (10 min of stack formation), nor were organelle rearrangements observed. Therefore, it can be interpreted that stack formation is at least initially driven by remodeling of ER membranes at several sites of pre-existing membranes until IDR interactions are satisfied. The formation of stacks has likely long-lasting effects on organelle physiology and metabolism, but other organelles contribute less to their initial formation. These findings offer an opportunity to use the 2-component system, hereafter called membrane stacker system, to alter the composition of the synthetic membrane compartment either by specifically recruiting endogenous ER-resident proteins, or alternatively membrane proteins ectopically expressed in the ER.

Therefore, it can be asked if synthetic membrane compartments could be applied to enrich specific proteins within or at the stacks. To test this, different endogenous membrane proteins, such as the translocon component Sec63, a protein of the glycosylation machinery Ost1, the P-type ATPase Pmr1, the TRP channel Yvc1, and the lipid enzymes Pis1 and Opi3 were tagged with the membrane stacker system. Not all of these proteins are natively located at the ER membrane, some were localized to the vacuolar or Golgi membranes. But all of these proteins formed coalescent foci with the Nsp1 FG-repeat component after blue light induction indicating their enrichment at these specific sites and possibly membrane clustering. Therefore, one may also use the membrane stacker system to incorporate non-ER-resident proteins from elsewhere in the cell by tagging them with an iLID domain, or alternatively, cluster or stack membranes of other organelles.

To establish that said approach can be used to enrich proteins within the synthetic membrane compartments, and thus to engineer a dedicated organelle, it was attempted to locally enrich several different membrane proteins. The ER membrane protein Spf1, a P-type ATPase and ion channel located in the ER membrane and transmembrane dislocase (McKenna, M. J. et al. The endoplasmic reticulum P5A-ATPase is a transmembrane helix dislocase. Science 369, (2020).), the vacuolar TRP homolog Yvc1 (Palmer, C. P. et al. A TRP homolog in Saccharomyces cerevisiae forms an intracellular Ca2+-permeable channel in the yeast vacuolar membrane. PNAS 98, 7801-7805 (2001).) and the Golgi membrane calcium ATPase Pmr1 (Antebi, A. & Fink, G. R. The Yeast Ca2+-ATPase Homologue, PMR1, is Required for Normal Golgi Function and Localizes in a Novel Golgi-like Distribution. Mol Biol Cell 3, 633-654 (1992).) were chosen and stably overexpressed to test for inducible condensation at membranes, in this case fused to one of the two genetic components. All of these proteins rapidly enriched within the respective foci as observed by fluorescence microscopy (Figure 4 c). Therefore, it is concluded that the composition of the synthetic membrane compartments can be genetically altered.

Next, the resulting membrane structures were targeted by cryo-ET and the resulting membrane morphology was assessed. Spf1 enriched membranes showed a similar membrane stacking phenotype as described above, where several stacked membrane sheets were located either at the cell cortex or the NE (Figure 4 c). However, stacked Spf1 membranes appeared more ruffled compared to the ones with a simple ER membrane helix, likely a consequence of the high number of Spf1 within the membrane. The vacuolar protein Yvc1 when overexpressed without induction was localized homogenously to the whole vacuolar membrane. The induction of condensation at these membranes by the system led to rapid clustering of Yvc1 at distinct sites that formed vacuolar membrane interfaces, which in cryo-EM presented as junctions between several vacuoles (Figure 4 c). When targeting these interfaces by cryo-ET it was possible to visualize smooth membranes a mean distance of 51.50 nm (SDEV = 9.94, n = 53 vacuole interfaces) consistent with the distance observed before in stacked membrane sheets. The membranes were enriched in proteins fitting the dimensions of the Yvc1 (Ahmed, T. et al. Structure of the ancient TRPY1 channel from Saccharomyces cerevisiae reveals mechanisms of modulation by lipids and calcium. Structure 30, 139-155.e5 (2022).). For the Golgi-localized ATPase Pmr1 (Antebi, A. & Fink, G. R. The Yeast Ca2+-ATPase Homologue, PMR1, is Required for Normal Golgi Function and Localizes in a Novel Golgi-like Distribution. Mol Biol Cell 3, 633-654 (1992).), no membrane stacking events were observed, despite the clear presence of coalescent foci in fluorescence (Figure 4 c). Instead, isolated circular Golgi membranes and vesicular clusters were observed, that also colocalized with a fluorescent PH-domain reporter. Taken together these results indicated that condensation of FG-repeats on membranes provides an effective way to artificially cluster native membranes into defined compartments, to enrich membranes proteins within these dynamically forming, synthetic membrane compartments but also to further fine-tune their properties.

The endomembrane system of S. *cerevisiae* is largely conserved to mammalian cells, but has a less complex architecture. To understand if similar principles of artificial membrane remodeling by condensation apply to mammalian cells, the system was transferred into stably transfected human embryonic kidney cells using the Flp-In T-REx system that contains a tet-operator for tetracycline-inducible expression (Figure 5 a). After induction of protein expression, cells showed ER localized fluorescence of the membrane anchoring component and largely dispersed cytosolic fluorescence for the intrinsically disordered component. After blue light induction, cells formed coalescent foci, that often cluster at a few distinct locations in the cell. Cryo-lamellae of these cells and targeted cellular membrane structures were prepared by cryo-ET. Besides classical Golgi membrane stacks and complex endomembrane structures, several instances of membrane stacking was found similar to what had been described here for S. *cerevisiae* cells. Therefore, it can be concluded that the induction of synthetic membrane compartments is possible in human cells and likely can be broadly employed.

## Claims

1. A system comprising
a component A comprising a membrane anchor, and
a component B comprising a multivalent protein or a multivalent peptide,
wherein either
i) the component A is capable to bind to the component B, or
ii) the component A and the component B form a compound in which component A is bound to component B.

2. The system according to claim 1, wherein the membrane anchor comprises a lipid binding domain, an amphipathic α-Helix, a hydrophobic transmembrane domain, a lipid anchor, a fatty acid chain, a peripheral membrane protein, an integral membrane protein, a lipid anchored protein, a transmembrane protein or the membrane or transmembrane domains thereof.

3. The system according to any of the preceding claims, wherein the membrane anchor comprises an organelle specific membrane anchor.

4. The system according to any of the preceding claims, wherein the membrane anchor comprises a nucleus specific membrane anchor, an endoplasmic reticulum (ER) specific membrane anchor, vacuole specific membrane anchor, endo-lysosomal membrane anchor, Golgi apparatus specific membrane anchor, chloroplast specific membrane anchor, peroxisome specific membrane anchor, an mitochondria specific membrane anchor, or a plasma membrane specific membrane anchor.

5. The system according to any of the preceding claims, wherein the membrane anchor comprises a membrane protein or the membrane or transmembrane domain thereof selected from an ABC (ATP-binding-cassette) transporter, a transmembrane ATPase, an ion channel, a solute carrier (SLC) transporter, an endoplasmic reticulum (ER)- transmembrane protein, an endo-lysosomal transmembrane protein, a protein translocase, a lipid enzyme, a lipid scramblase, lipid transporter, a plasma membrane surface receptor, a cell adhesion protein, or a G-protein coupled receptor.

6. The system according to any of the preceding claims, wherein the multivalent protein or the multivalent peptide comprises a phase separating protein or phase separating peptide.

7. The system according to any of the preceding claims, wherein the multivalent protein or the multivalent peptide comprises intrinsically disordered proteins (IDP), intrinsically disordered protein regions (IDR), or multidomain proteins having one or more multiple protein interactions domains.

8. The system according to any of the preceding claims, wherein the multivalent protein or the multivalent peptide comprises fused in Sarcoma (FUS), FUSn, or fragments thereof, FG-repeats, the heterogeneous nuclear ribonucleoprotein A1 (hnRNPA1) or fragments thereof, a combination of one or more Sumo domains and Sumo interaction motifs, or a combination of one or more SH3 domains and proline-rich motifs.

9. The system according to any of the preceding claims, wherein the system is inducible.

10. The system according to any of the preceding claims, wherein component A and component B further comprise one or more protein interaction domains (PID), wherein the PIDs of component A are ligands of the PIDs of component B.

11. The system according to any of the preceding claims, wherein the PIDs of component A and component B comprise protein dimerizing domains of a protein dimerizing system.

12. The system according to any of the preceding claims, further comprising a component C, wherein component C comprises proteins, RNAs, lipids, or small molecules.

13. A component A comprising a membrane anchor, wherein either
i) the component A is capable to bind to a component B comprising a multivalent protein or a multivalent peptide, or wherein
ii) the component A and the component B form a compound in which the component A is bound to the component B.

14. A kit of parts comprising
a component A comprising a membrane anchor, and
a component B comprising a multivalent protein or a multivalent peptide,
wherein either
the component A is capable to bind to the component B, or
the component A and the component B form a compound in which component A is bound to component B.

15. Nucleic acid molecules comprising nucleotide sequences encoding the components and/or compounds according to any of the preceding claims.

16. A cell comprising the system according to any of claims 1 to 12, the component A according to claim 13, the kit of parts according to claim 14 or the nucleic acid molecules according to claim 15.

17. A method for producing artificial membrane compartments comprising introducing the system according to any of claims 1 to 12, the component A according to claim 13, the kit of parts according to claim 14, or the nucleic acid molecule according to claim 14 into cells or cell lines, culturing the cells or cell lines to express the components or compounds of the system, or the nucleic acid molecule.

18. Artificial membrane compartments and/or artificial membrane compartment-specific membranes obtainable by the method of claim 17.

19. A composition or pharmaceutical composition comprising the artificial membrane compartments and/or artificial membrane compartment-specific membranes of claim 18.

20. Artificial membrane compartments and/or artificial membrane compartment-specific membranes according to claim 18 or pharmaceutical composition according to claim 19 for use in therapy.
